(19) Europäisches Patentamt
European Patent Office
Office européen des brevets

(11) EP 3 954 994 B1

(12) # EUROPÄISCHE PATENTSCHRIFT

(45) Veröffentlichungstag und Bekanntmachung des Hinweises auf die Patenterteilung:
**30.04.2025 Patentblatt 2025/18**

(21) Anmeldenummer: **20190257.4**

(22) Anmeldetag: **10.08.2020**

(51) Internationale Patentklassifikation (IPC):
***G01N 33/52*** (2006.01) ***G01N 33/84*** (2006.01)

(52) Gemeinsame Patentklassifikation (CPC):
**G01N 33/52; G01N 33/84**

(54) **REAGENZ ZUR BESTIMMUNG VON AMMONIUM-IONEN IN WÄSSRIGER LÖSUNG**

REAGENT FOR DETECTING AMMONIUM IONS IN AQUEOUS SOLUTION

RÉACTIF DESTINÉ À LA DÉTERMINATION DES IONS D'AMMONIUM EN SOLUTION AQUEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.02.2022 Patentblatt 2022/07**

(73) Patentinhaber: **AXAGARIUS GmbH & Co. KG
52355 Düren (DE)**

(72) Erfinder:
- **DICKSCHAT, Arne
50321 Brühl (DE)**
- **HOFFMANN, Jürgen
52355 Düren (DE)**

(74) Vertreter: **dompatent von Kreisler Selting Werner -
Partnerschaft von Patent- und Rechtsanwälten mbB
Deichmannhaus am Dom
Bahnhofsvorplatz 1
50667 Köln (DE)**

(56) Entgegenhaltungen:
**EP-A1- 0 707 210 WO-A1-99/61892**

- **ROSE H. BROWN ET AL: "A colorimetric micromethod for determination of ammonia; the ammonia content of rat tissues and human plasma", ARCHIVES OF BIOCHEMISTRY AND BIOPHYSICS, vol. 66, no. 2, 1 February 1957 (1957-02-01), US, pages 301 - 309, XP055756708, ISSN: 0003-9861, DOI: 10.1016/S0003-9861(57)80005-8**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 954 994 B1

## Beschreibung

### Technisches Gebiet

Die Erfindung betrifft ein feststoffliches Reagenz zur Bestimmung von Ammonium-Ionen ($NH_4^+$, Ammoniumkation) in wässriger Lösung, ein Verfahren zu dessen Herstellung, ein Verfahren zum qualitativen Nachweis von Ammonium-Ionen in wässriger Lösung sowie ein Verfahren zur quantitativen Bestimmung von Ammonium-Ionen in wässriger Lösung unter Verwendung des feststofflichen Reagenzes.

### Hintergrund der Erfindung

Die Bestimmung des Ammonium-Ionengehaltes einer wässrigen Lösung ist eine wichtige Routineaufgabe der Umwelt- bzw. Wasseranalytik geworden. Die Bestimmung der Konzentration von Ammonium-Ionen mittels der Berthelot-Reaktion ist besonders zuverlässig aufgrund der hohen Nachweisempfindlichkeit, der hohen Selektivität und der relativ geringen Störanfälligkeit. Dabei sind die Anwendungsgebiete vielfältig. So ist es möglich die Ammonium-Ionenkonzentration in Wasser, Lebensmitteln, Bodenextrakten oder biologischen Materialien zuverlässig zu bestimmen.

Die Berthelot-Reaktion, die auch als Indophenol-Reaktion bekannt ist, ist die Bezeichnung für die Reaktion zwischen Ammonium-Ionen und einem Phenol, welche unter geeigneten oxidierenden Bedingungen zur Bildung eines Indophenol-Farbstoffes (der eine starke Absorption zwischen 630 und 720 nm aufweist) führt. Der Reaktionsmechanismus ist komplex und noch nicht vollständig aufgeklärt. Es wird jedoch vermutet, dass im ersten Schritt die Ammonium-Ionen durch Erhöhung des pH-Wertes zu Ammoniak deprotoniert werden, welcher mit dem Hypohalit reagiert (im Nachfolgenden beispielhaft für Hypochlorit gezeigt). Das *in situ* generierte Monochloramin wird anschließend unter Verwendung eines Katalysators (z. B. Nitroprussidnatrium (NPN)) mit zwei Molekülen einer Phenolverbindung (im Nachfolgenden beispielhaft für Phenol gezeigt) zu einem Indophenol-Farbstoff umgesetzt (oxidativer Dehydrogenierungsprozess). Um eine erfolgreiche Reaktion zu gewährleisten, muss ein verhältnismäßiger hoher pH-Wert (pH > 10,5) eingestellt werden. Die angesprochenen Schritte der Reaktion sind im nachfolgenden Reaktionsschema dargestellt:

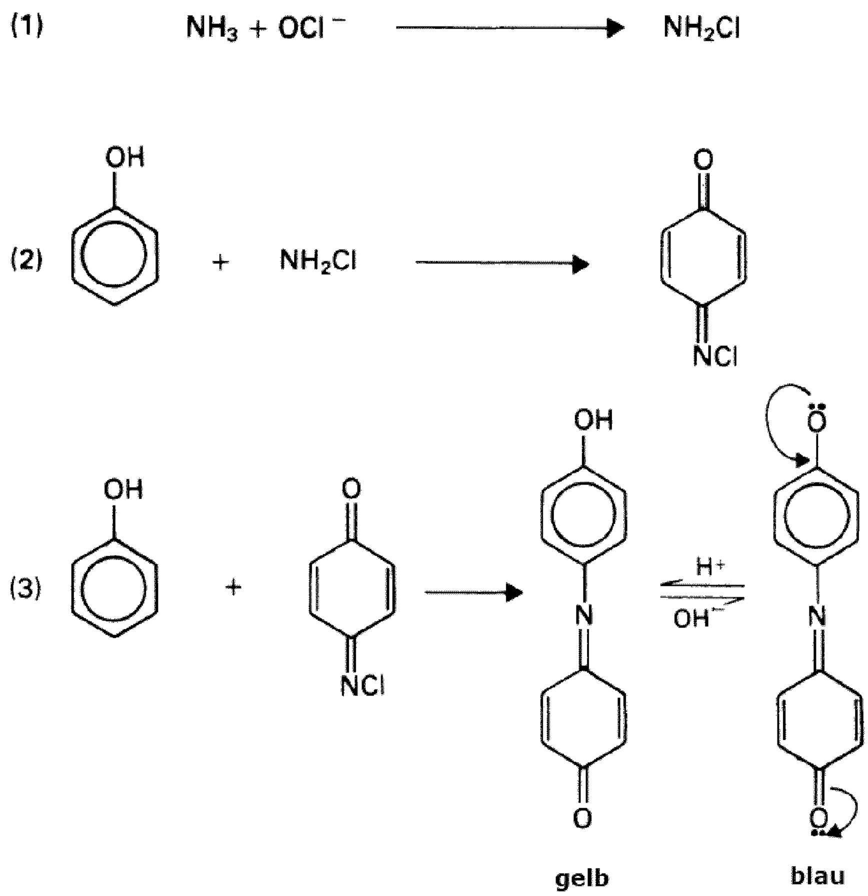


*Abb. 1 Vermutetes Reaktionsschema der Berthelot-Reaktion*

***Stand der Technik***

EP 707210 betrifft Mittel und Verfahren zur Bestimmung von Ammonium-Ionen in wässrigen Lösungen auf Grundlage der Berthelot-Reaktion. Hierbei wird ein mit einem Phenolderivat imprägnierter Träger mit Hypochlorit, Hypobromit oder einer Hypohalitbildner-enthaltenden und mit Natronlauge alkalisch gemachten Probelösung für einige Zeit in Kontakt gebracht. Die Verfärbung des Trägers gibt Aufschluss über die Anwesenheit von Ammonium-Ionen. Hierbei umfasst der Träger neben dem Phenolderivat zusätzlich noch Katalysatoren und Puffersubstanzen, wobei die Probelösung ebenfalls mit Puffersubstanzen versetzt sein kann.

EP 1840566 betrifft einen Aufbau bzw. eine Anordnung zur Bestimmung des Ammonium-Ionengehalts einer flüssigen Probe. Diese Anordnung weist ein Indikator-Element auf, welches ein Indikator-Gemisch aus Ammonium-Chlorid und einen pH-Indikator umfasst. Das beschriebene Indikator-Gemisch ist gegenüber der umgebenden Atmosphäre isoliert, so dass das Atmosphären-Gas nicht in das Indikator-Gemisch eindringen kann. Zwischen dem pH-Indikator-Gemisch und der Flüssigkeitsprobe weist die Anordnung eine gasdurchlässige Membran auf. Die Bestimmung des Farbumschlags erfolgt durch ein auf das Indikator-Element gerichtetes Fotometer. Die Funktionsweise der Anordnung wird wie folgt beschrieben: "*Zu Beginn einer Messung diffundiert zunächst der Wasserdampf aus der Flüssigkeitsprobe durch die gasdurchlässige Membran in das Indikator-Gemisch hinein, wobei der Wasserdampf das Indikator-Gemisch auflöst, bis dessen Dampfdruck dem der Flüssigkeitsprobe entspricht. Danach bleibt der Wasseranteil in dem Indikator-Gemisch konstant. Das durch die gasdurchlässige Membran in das Indikator-Gemisch diffundierende Ammoniak-Gas verschiebt den pH-Wert der Ammonium-Chlorid-Lösung.* "

WO 2018/054797 betrifft ein Verfahren zur Bestimmung von Ammonium bzw. Ammoniak in wässrigen Proben unter Verwendung eines ZweikomponentenSystems für die Berthelot-Reaktion. Zur quantitativen und qualitativen Auswertung wird die blaue Farbe des entstandenen Indophenols photometrisch gemessen. Das beschriebene Verfahren soll neben dem Messergebnis einer einzelnen Messung auch direkt Informationen zur Plausibilität dieses Messergebnisses liefern, indem neben der Messung der Extinktion im Absorptionsbereich des gebildeten blauen Indolfarbstoffes bei der Probe zusätzlich eine Messung der Extinktion im Absorptionsbereich des als Katalysator eingesetzten Nitroprussids

vorgenommen wird.

EP 1566632 betrifft einen Teststreifen, mit welchem in einem Schritt (sog. "*dip and read*") die Ammonium-Ionenkonzentration in wässrigen Lösungen bestimmt werden kann und bei dem sich alle erforderlichen Reagenzien in einem einzigen Pad befinden. Die Detektion erfolgt mittels Farbumschlag von pH-Indikatoren.

Die DIN ISO 15923-1:2013 betrifft die Bestimmung von Ammonium in wässrigen Lösungen mittels Berthelot-Reaktion mit anschließender photometrischer Detektion. Hierzu werden zwei Reagenzien bereitgestellt, die mit der Probelösung vermischt werden. Das Nitroprussidnatrium-Reagenz ist eine wässrige Lösung aus Natriumsalicylat, Natriumcitrat ("*um Störungen durch Kationen, wie Calcium- oder Magnesium-Ionen zu maskieren*") und Nitroprussidnatrium. Das zweite Reagenz ist eine wässrige Lösung aus Natriumhydroxid und Natriumdichlorisocyanurat (Dichlorisocyanurat-Reagenz oder auch DIC-Reagenz genannt). Beide Reagenzien werden zu jeweils einem Volumenanteil mit bis zu 10 Volumenanteilen der Probelösung vermischt und bei 30 bis 40 °C für wenigstens 480 Sekunden inkubiert bevor im Anschluss die Extinktion bei 660 nm gemessen wird. Der Kalibrierbereich für dieses standardisierte Verfahren liegt bei 0,05 bis 2,0 mg/L.

## *Aufgabe*

Herkömmliche, im Stand der Technik bekannte Verfahren zur Bestimmung von Ammonium-Ionen in wässriger Lösung, einschließlich der obengenannten DIN-Vorschrift, welche auf der Berthelot-Reaktion beruhen, verwenden wenigstens zwei unterschiedliche Reagenzien. Dies führt jedoch dazu, dass diese Verfahren eine Vielzahl von Arbeitsschritten erfordern. Es besteht daher ein Bedarf an einem verbesserten Verfahren zur Bestimmung der Ammonium-Ionenkonzentration in wässriger Lösung, das sowohl den Arbeits-, als auch den Materialaufwand reduziert.

Bei gängigen "ready-to-use"-Testkits muss zudem der störende Einfluss der Probenmatrix berücksichtigt werden. Ein weiteres Problem bei der Bestimmung von Ammonium-Ionen in wässriger Lösung mittels der Berthelot-Reaktion ist, dass die besagte Reaktion stark abhängig ist von der Konzentration des Chlorspenders, sodass dieser üblicherweise zum Ende hinzugegeben wird um möglichst eine vollständige Chlorierung von $NH_3$ zu Monochloramin zu gewährleisten und, falls eine zu hohe Konzentrationen des Chlorspenders verwendet wurde, wird der Indophenol-Farbstoff oxidiert, was zu Unterbefunden führt. "Ready-to-use"-Testkits unterliegen einer Alterung auf Grund welcher es zu Unterbefunden, aber auch zu Überbefunden kommen kann.

Erfindungsgemäß wurde gefunden, dass das der Erfindung zugrundeliegende Problem dadurch gelöst wird, dass alle für die Berthelot-Reaktion notwendigen Substanzen in einem einzigen feststofflichen Reagenz (Einkomponenten-System) vorliegen. Dabei wurde jedoch beobachtet, dass die Substanzen, die üblicherweise im Rahmen der Berthelot-Reaktion Anwendung finden, nicht als Feststoffe kombinierbar sind. Als besonders kritisch für die Haltbarkeit des Reagenzes erwies sich dabei die Wahl der Base. Hierbei wurde experimentell festgestellt, dass für die Wahl einer geeigneten Base eine Vielzahl von Kriterien berücksichtigt werden muss und dass die erfindungsgemäß eingesetzte Base eine starke Base sein sollte, die unter Standard-Lagerbedingungen nicht nur wenig hygroskopisch ist, sondern auch gleichzeitig gegenüber der im Gemisch vorhandenen Hypohalitquelle oxidationsbeständig sein sollte.

## Kurzbeschreibung der Erfindung

Die der Erfindung zugrundeliegende Aufgabe wurde gelöst durch die Merkmale der unabhängigen Ansprüche 1, und 11 bis 14.

Der erste Aspekt der Erfindung betrifft dabei ein feststoffliches Reagenz zur Bestimmung von Ammonium-Ionen in wässriger Lösung umfassend oder im Wesentlichen bestehend aus einem Gemisch der nachfolgenden Komponenten:

(i) Katalysator,
(ii) Base,
(iii) Hypohalitquelle, und
(iv) Phenolverbindung,

wobei der Katalysator Nitroprussidnatrium oder Natriumhexacyanoferrat ist;
wobei die Base einen $pK_B$-Wert von -2 bis 4 aufweist mit der Maßgabe, dass die Base kein Hydroxid ist; und
wobei die Phenolverbindung ein ortho- und/oder meta-substituiertes Phenol gemäß der folgenden Struktur ist:

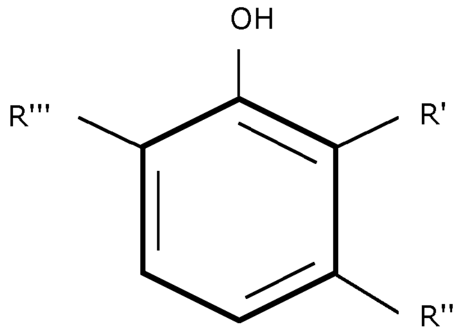

wobei die Substituenten R', R" und R‴ unabhängig voneinander ausgewählt sind aus der Gruppe bestehend aus H, Alkyl, Aryl, $CO_2H$, $CO_2Li$, $CO_2Na$, $CO_2K$, CO, $C(O)NAlkyl_2$, $C(O)NH_2$, C(S)OAlkyl, C(O)SAlkyl, $CO_2Alkyl$, OAlkyl, SAlkyl, $CH(OAlkyl)_2$, CH(OAlkyl)(SAlkyl), $CH(SAlkyl)_2$, F, Cl, Br, I, OH und SH;
wobei Alkyl eine Kohlenwasserstoffgruppe der allgemeinen Formel $C_nH_{2n+1}$ ist, wobei "n" der Anzahl der Kohlenstoffatome entspricht und ein ganzzahliger Wert im Bereich von 1 bis 18 ist; und
Aryl eine aromatische Kohlenstoffgruppe mit 5 bis 10 Kohlenstoffen ist.

In einer Ausführungsform des vorstehend beschriebenen feststofflichen Reagenzes ist der Katalysator Nitroprussidnatrium.

In einer weiteren Ausführungsform des vorstehend beschriebenen feststofflichen Reagenzes ist die Base ausgewählt aus der Gruppe bestehend aus Alkali- und/oder Erdalkalimetallphosphaten, Alkali- und/oder Erdalkalimetallcarbonaten und Alkali- und/oder Erdalkalimetalloxiden.

In einer weiteren Ausführungsform des vorstehend beschriebenen feststofflichen Reagenzes ist die Base ausgewählt ist aus der Gruppe bestehend aus Alkalimetallphosphaten, Alkalimetallcarbonaten und Erdalkalimetalloxiden.

In einer weiteren Ausführungsform des vorstehend beschriebenen feststofflichen Reagenzes ist die Hypohalitquelle ausgewählt aus der Gruppe bestehend aus Dichlorisocyanursäure, Trichlorcyanursäure und/oder deren Salze.

In einer weiteren Ausführungsform des vorstehend beschriebenen feststofflichen Reagenzes ist die Phenolverbindung ausgewählt aus der Gruppe bestehend aus Phenol, Thymol, Salicylsäure und/oder deren Salze, $\alpha$-Naphthol, Guajacol, o-Phenylphenol, o-Chlorphenol, 2-Methyl-5-hydroxychinolin und m-Kresol.

In einer weiteren Ausführungsform des vorstehend beschriebenen Feststofflichen Reagenzes umfasst das feststoffliche Reagenz mindestens 2 Gew.% an Base in Bezug auf das Gesamtgewicht des getrockneten Reagenzes und/oder mindestens 2 Gew.% an Phenolverbindung in Bezug auf das Gesamtgewicht des getrockneten Reagenzes.

In einer weiteren Ausführungsform des vorstehend beschriebenen feststofflichen Reagenzes umfasst das feststoffliche Reagenz

- bis zu 1 Gew.% an (i), bis zu 2,5 Gew.% an (ii), bis zu 1 Gew.% an (iii) und bis zu 25 Gew.% an (iv) in Bezug auf das Gesamtgewicht des getrockneten Reagenzes,
- bis zu 1 Gew.% an (i), bis zu 5 Gew.% an (ii), bis zu 1 Gew.% an (iii) und bis zu 25 Gew.% an (iv) in Bezug auf das Gesamtgewicht des getrockneten Reagenzes,
- bis zu 0,5 Gew.% an (i), bis zu 55 Gew.% an (ii), bis zu 0,5 Gew.% an (iii) und bis zu 15 Gew.% an (iv) in Bezug auf das Gesamtgewicht des getrockneten Reagenzes, oder
- bis zu 1 Gew.% an (i), bis zu 20 Gew.% an (ii), bis zu 0,75 Gew.% an (iii) und bis zu 20 Gew.% an (iv) in Bezug auf das Gesamtgewicht des getrockneten Reagenzes umfasst, wobei jeweils der Rest aus Additiven besteht.

In einer weiteren Ausführungsform des vorstehend beschriebenen feststofflichen Reagenzes ist das Reagenz frei von Wasser.

In einer weiteren Ausführungsform des vorstehend beschriebenen Feststofflichen Reagenzes liegt das Reagenz als Pulverkissen vor.

Das feststoffliche Reagenz kann in Tablettenform vorliegen.

Der zweite Aspekt der Erfindung betrifft ein Testkit zur Bestimmung von Ammonium-Ionen in wässriger Lösung umfassend das feststoffliche Reagenz gemäß dem ersten Aspekt der Erfindung.

Der dritte Aspekt der Erfindung betrifft ein Verfahren zum qualitativen Nachweis von Ammonium-Ionen in wässriger Lösung umfassend die folgenden Schritte:

a) Bereitstellen einer Probelösung;

b) Zugabe des feststofflichen Reagenzes gemäß dem ersten Aspekt der Erfindung zur vorgelegten Probelösung oder Vorlegen des feststofflichen Reagenzes und anschließende Zugabe der Probelösung;
c) Rühren der erhaltenen Lösung;
d) Inkubation unter Normalbedingungen für 30 min oder bis eine Verfärbung der Lösung eingetreten ist.

In einer bevorzugten Ausführungsform dieses Verfahrens zum qualitativen Nachweis von Ammonium-Ionen in wässriger Lösung werden für pro Milliliter Probelösung 10 bis 40 mg und bevorzugt 15 bis 30 mg des feststofflichen Reagenzes verwendet.

Der vierte Aspekt der Erfindung betrifft ein Verfahren zur quantitativen Bestimmung von Ammonium-Ionen in wässriger Lösung umfassend die folgenden Schritte:

a) Bereitstellen einer Probelösung;
b) Pipettieren der Probelösung in eine Küvette;
c) Zugabe des feststofflichen Reagenzes gemäß dem ersten Aspekt der Erfindung zur Probelösung;
d) Vollständiges oder teilweises Auflösen des zugegebenen feststofflichen Reagenzes;
e) Inkubation unter Normalbedingungen für 30 min;
f) Photometrische Extinktionsbestimmung der Lösung bei einer Wellenlänge von 680 nm.

In einer bevorzugten Ausführungsform dieses Verfahrens zum qualitativen Nachweis von Ammonium-Ionen in wässriger Lösung werden für pro Milliliter Probelösung 10 bis 40 mg und bevorzugt 15 bis 30 mg des feststofflichen Reagenzes verwendet.

Der fünfte Aspekt der Erfindung betrifft die Verwendung des feststofflichen Reagenzes gemäß dem ersten Aspekt der Erfindung zum qualitativen Nachweis und/oder der quantitativen Bestimmung von Ammonium-Ionen in wässriger Lösung.

Das erfindungsgemäße Reagenz bringt eine Reihe von Vorteilen mit sich. So ermöglicht das erfindungsgemäße Reagenz, unter anderem, ein schnelles und unkompliziertes Analyseverfahren für die Bestimmung von Ammonium-Ionen in wässriger Lösung, indem es die Verwendung mehrerer Lösungen, wie es bei den im Stand der Technik bekannten Verfahren zur Bestimmung von Ammonium-Ionen in wässriger Lösung üblich ist, überflüssig macht. Im Zuge dieser Verbesserung wird auch der Materialaufwand reduziert. Dabei weist das Reagenz eine hohe Lagerstabilität auf, die sich dadurch auszeichnet, dass sich selbst bei langer Lagerung (sogar bei Lagerung unter Stressbedingungen) die Nachweisgrenze und die Bestimmungsgrenze des auf der Berthelot-Reaktion beruhenden Analyseverfahrens nicht verschlechtert.

**Beschreibung der Figuren**

Figur 1 zeigt eine Kalibrierung für die quantitative Bestimmung der Ammonium-Ionenkonzentration mittels Reagenz 1. Das erfindungsgemäße Verfahren zur quantitativen Bestimmung von Ammonium-Ionen in wässriger Lösung wurde für Proben bekannter Ammonium-Ionenkonzentration (0,1, 0,2, 0,3, 0,4 und 0,5 mg/L, siehe entsprechende Punkte) angewandt. Die gepunktete Linie zeigt die Kalibrierungskurve und die zugehörige Fitfunktion ist ebenfalls gezeigt.

Figur 2 zeigt die Auswertung eines Lagerversuchs für Reagenz 1 unter Stressbedingungen. Die gestrichelte Linie entspricht dem Sollwert der Standardlösung von 0,5 mg/L, die in regelmäßigen Abständen mit dem erfindungsgemäßen Verfahren zur quantitativen Bestimmung von Ammonium-Ionen analysiert wurde. Die gepunkteten Linien zeigen die Fehlertoleranz von $\pm$ 0,05 mg/L.

Figur 3 zeigt ein Extinktionsspektrum des Indophenolblaus, welches nach der Berthelot-Reaktion mit Salicylsäure als Phenolverbindung erhalten wurde.

Figur 4 zeigt eine Kalibrierung für die quantitative Bestimmung der Ammonium-Ionenkonzentration mittels Reagenz 2. Das erfindungsgemäße Verfahren zur quantitativen Bestimmung von Ammonium-Ionen in wässriger Lösung wurde für Proben bekannter Ammonium-Ionenkonzentration angewandt. Gezeigt sind die Messpunkte, die Kalibrierungskurve und die zugehörige Fitfunktion.

Figur 5 zeigt die Auswertung eines Lagerversuchs für Reagenz 3 unter Stressbedingungen. Die gestrichelte Linie entspricht dem Sollwert der Standardlösung von 0,5 mg/L, die in regelmäßigen Abständen mit dem erfindungsgemäßen Verfahren zur quantitativen Bestimmung von Ammonium-Ionen analysiert wurde. Die gepunkteten Linien zeigen die Fehlertoleranz von ± 0,05 mg/L.

Figur 6 zeigt die Auswertung eines Lagerversuchs für Reagenz 4 unter Stressbedingungen. Die gestrichelte Linie entspricht dem Sollwert der Standardlösung von 0,5 mg/L, die in regelmäßigen Abständen mit dem erfindungsgemäßen Verfahren zur quantitativen Bestimmung von Ammonium-Ionen analysiert wurde. Die gepunkteten Linien zeigen die Fehlertoleranz von ± 0,05 mg/L.

Figur 7 zeigt eine Kalibrierung für die quantitative Bestimmung der Ammonium-Ionenkonzentration mittels Reagenz 5. Das erfindungsgemäße Verfahren zur quantitativen Bestimmung von Ammonium-Ionen in wässriger Lösung wurde für Proben bekannter Ammonium-Ionenkonzentration angewandt. Gezeigt sind die Messpunkte, die Kalibrierungskurve und die zugehörige Fitfunktion.

Figur 8 zeigt eine Kalibrierung für die quantitative Bestimmung der Ammonium-Ionenkonzentration mittels Reagenz 6. Das erfindungsgemäße Verfahren zur quantitativen Bestimmung von Ammonium-Ionen in wässriger Lösung wurde für Proben bekannter Ammonium-Ionenkonzentration angewandt. Gezeigt sind die Messpunkte, die Kalibrierungskurve und die zugehörige Fitfunktion.

Figur 9 zeigt eine Kalibrierung für die quantitative Bestimmung der Ammonium-Ionenkonzentration mittels Reagenz 7. Das erfindungsgemäße Verfahren zur quantitativen Bestimmung von Ammonium-Ionen in wässriger Lösung wurde für Proben bekannter Ammonium-Ionenkonzentration angewandt. Gezeigt sind die Messpunkte, die Kalibrierungskurve und die zugehörige Fitfunktion.

**Begriffsdefinitionen**

Das Ammonium-Ion (mit den Synonymen: $NH_4^+$, Ammonium oder Azanium-Ion) ist die konjugierte Säure zur Base Ammoniak ($NH_3$). Es handelt sich hierbei um ein Kation, das mit Anionen Salze bilden kann.

Der Begriff "Hydroxid" ist eingeschränkt auf Metallhydroxide (Alkalimetallhydroxide, Erdalkalimetallhydroxide, Übergangsmetallhydroxide und die Hydroxide der Metalle der dritten Hauptgruppe), d. h. solche Verbindungen, die abdissoziierbare Hydroxid-Ionen ($OH^-$) enthalten. Es handelt sich hierbei nicht um die sauren Verbindungen (Alkohole, Carbonsäuren, etc.) die lediglich eine Hydroxy-Gruppe tragen.

Sofern nicht anders angegeben, werden alle Reaktionen und Verfahren (z. B. Bestimmungen oder Messmethoden) unter Normalbedingungen, d. h. bei der Standardtemperatur von 25 °C und beim Standarddruck von 101,3 kPa, durchgeführt.

Gemäß der vorliegenden technischen Lehre ist unter dem Begriff "feststofflich" zu verstehen, dass die damit gekennzeichnete Substanz unter Normalbedingungen in einem festen Aggregatzustand, d. h. als Feststoff, vorliegt. Diese Eigenschaft ist unabhängig davon, ob der besagte Stoff organischer oder anorganischer Natur ist.

Ein Salz ist eine feststoffliche Verbindung, die aus einer Anordnung von Kationen und Anionen besteht. Unter einem Salz einer Säure oder Base ist zu verstehen, dass es sich um die deprotonierte Säure (Anion) bzw. die protonierte Base (Kation) handelt, die zusammen mit einem entsprechenden Gegenion ein Kristallgitter bilden. Das Gegenion zur deprotonierten Säure kann ein Alkali- oder Erdalkalimetallion sein; auch Ionen der Übergangsmetalle (z. B. in wässriger Lösung unter Normalbedingungen stabile Kationen von Chrom, Mangan, Eisen, Cobalt, Kupfer oder Silber) sind als Gegenion denkbar.

Unter "Stabilität" wird Lagerstabilität bei Lagerung unter Normalbedingungen verstanden.

Die Einheit der Konzentration, sofern nicht anders angegeben, ist [g/mL], mit der Maßgabe, dass die Einheit der Konzentration für den Bestimmungsbereich von Ammonium [mg/L] ist.

Der Begriff "umfassen" schließt, außer seiner wörtlichen Bedeutung, auch die Ausdrücke "bestehen im Wesentlichen aus" und "bestehen aus" mit ein. Somit kann Gegenstand, der speziell aufgelisteten Elemente "umfasst" neben diesen Elementen weitere Elemente enthalten oder im in Sinne von "bestehen aus" keine weiteren Elemente enthalten. Die Begriffe "aufweisen", "beinhalten" und "enthalten" schließen weitere Elemente oder Schritte nicht aus. Der Ausdruck "bestehen im Wesentlichen aus" bedeutet, dass die essentiellen Komponenten einer Zusammensetzung bzw. Schritte eines Verfahrens aufgelistet sind, die Zusammensetzung bzw. das Verfahren jedoch weitere (nicht aufgelistete) Bestandteile bzw. Schritte beinhalten kann, die die grundlegenden (Material-) Eigenschaften der Zusammensetzung bzw. des Verfahrens nicht wesentlich beeinflussen.

Die Verwendung des unbestimmten Artikels schließt eine Mehrzahl nicht aus.

Spezifität ist die Fähigkeit eines Verfahrens, eine Substanz oder eine Substanzklasse ohne Verfälschung durch andere in der Probe vorhandene Komponenten zu erfassen und sie somit eindeutig zu identifizieren.

Als Sensitivität (Empfindlichkeit) eines Verfahrens wird die Stärke der Änderung in der Antwort eines Messsignals geteilt durch die Änderung der auslösenden Größe (z. B. der Zielanalytkonzentration) bezeichnet. Die Sensitivität einer analytischen Methode entspricht der Steigung der Kalibrierkurve.

Die Messpräzision ist ein Maß für die Schwankungen, die durch die Testvorrichtung oder dem damit verbundenen Analysengerät verursacht werden. Sie wird durch die Mehrfachanalyse eines Standards ermittelt (sofern nicht anders angegeben, wird der Standard sechsfach ermittelt).

Die Methodenpräzision ist die zufällige Streuung der Analysenergebnisse. Sie wird durch eine mehrfache Durchführung der gesamten Analyse, d. h. vom Abwiegen über die Probenvorbereitung bis zu der Messung und Befund ermittelt (sofern nicht anders angegeben, werden die Analysen in sechsfach durchgeführt).

Die Nachweisgrenze eines Verfahrens ist die kleinste Konzentration des Analyten in einer Probe, die qualitativ (aber nicht quantitativ) erfasst werden kann (Ja/Nein-Entscheidung).

Die Bestimmungsgrenze eines Verfahrens ist die kleinste Konzentration des Analyten in der Probe, die mit gegebener Präzision und Richtigkeit quantitativ bestimmt werden kann.

Gemäß der erfindungsgemäßen Technologie umfasst Begriff "Bestimmung" neben dem qualitativen Nachweis auch die quantitative Bestimmung der Konzentration des Analyten. Gemäß der erfindungsgemäßen Technologie umfasst Begriff "Nachweis" den qualitativen Nachweis aber nicht die quantitative Bestimmung der Konzentration des Analyten.

Die Formulierung "mehrere" bestimmt die ganzzahlige Anzahl der Sache, auf die sich die Formulierung bezieht, wobei 0 und 1 ausgeschlossen sind. "Mehrere" bedeutet, dass eine Anzahl von 2, 3, 4, 5, 6, 7, 8, 9 oder 10 bis 100 einer bestimmten Sache vorliegen kann.

Gemäß der vorliegenden technischen Lehre ist das Alkalimetall-Gegenion ausgewählt aus der Gruppe bestehend aus Natrium, Kalium und Lithium. Die bevorzugten Alkalimetall-Gegenionen sind Natrium oder Lithium. Ganz besonders bevorzugt ist, dass Natrium das Alkalimetall-Gegenion ist. Gemäß der vorliegenden technischen Lehre ist das Erdalkalimetall-Gegenion ausgewählt aus der Gruppe bestehend aus Magnesium, Calcium, Strontium und Barium. Die bevorzugten Erdalkalimetall-Gegenionen sind Magnesium, Calcium oder Strontium. Ganz besonders bevorzugt ist, dass das Erdalkalimetall-Gegenion Magnesium oder Calcium ist. Insbesondere bevorzugt ist, dass das Erdalkalimetall-Gegenion Calcium ist.

Gemäß der vorliegenden technischen Lehre entspricht die Lagerung unter Normalbedingungen einer Lagerung in einem trockenen, sauberen, gut belüfteten Raum bei einer Raumtemperatur zwischen 15 °C und 25 °C bzw. bis zu 30 °C in Abhängigkeit von externen klimatischen Bedingungen.

Bei einem Pulverkissen handelt es sich um eine in einer Folie oder in einem Film verpackte (z. B. in Aluminiumbeutel) Menge des Reagenzes, wobei das Pulverkissen 13 bis 17 mg, 26 bis 32 mg, 52 bis 64 mg, 78 bis 96 mg, 104 bis 128 mg, 130 bis 160 mg, 156 bis 192 mg oder bis zu 600 mg des erfindungsgemäßen feststofflichen Reagenzes umfassen kann.

## Detaillierte Beschreibung der Erfindung

### *Bestandteile des Reagenzes*

*Katalysator*

Katalysatoren sind ein Bestandteil des erfindungsgemäßen Reagenzes. Gemäß der vorliegenden technischen Lehre ist der Katalysator Natriumhexacyanoferrat oder Nitroprussidnatrium. Gemäß der vorliegenden technischen Lehre ist der Katalysator bevorzugt Nitroprussidnatrium.

Nitroprussidnatrium hat die folgende chemische Strukturformel:

$$Na_2[Fe(CN)_5NO]$$

und kann im Kristallgitter Wassereinschlüsse enthalten. Nitroprussidnatrium ist der meistverwendete Katalysator für die Berthelot-Reaktion. Obwohl der Reaktionsmechanismus nicht vollständig aufgeklärt ist, wird vermutet, ohne den erfindungsgemäßen Gegenstand zu beschränken, dass der Katalysator das Monochloramin stabilisiert und die Reaktion zwischen Monochloramin und der Phenolverbindung, d. h. den oxidativen Dehydrogenierungsprozess, katalysiert.

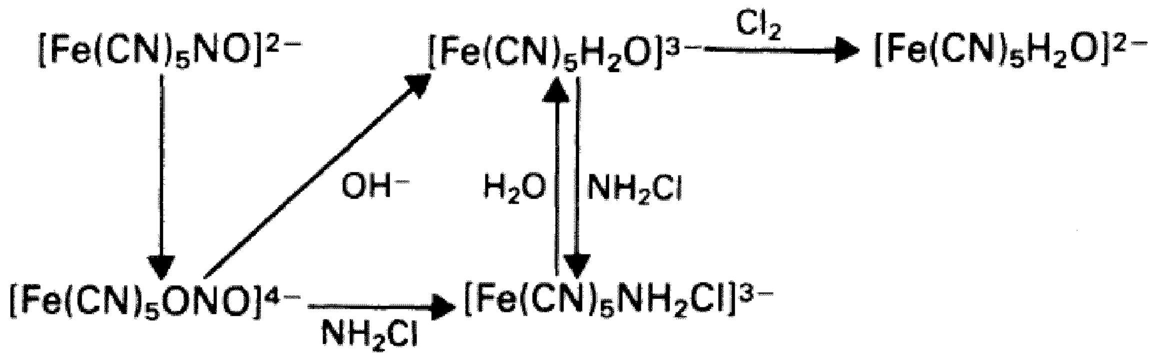


*Abb. 2. Stabilisierung des Monochloramin durch Nitroprussid*

*Base*

Gemäß der vorliegenden technischen Lehre kann die Base einen $pK_B$-Wert von -2 bis 4 haben mit der Maßgabe, dass die Base kein Hydroxid ist. In einer Bevorzugten Ausgestaltungsform hat die Base einen $pK_B$-Wert von 0,35 bis 3,75.

Gemäß der vorliegenden technischen Lehre ist die Base bei Lagerung unter Normalbedingungen wenig oder nicht hygroskopisch.

Gemäß der vorliegenden technischen Lehre ist die Base ausgewählt aus der Gruppe bestehend aus den Alkali- und/oder Erdalkalimetallphosphaten, Alkali- und/oder Erdalkalimetallcarbonaten und Alkali- und/oder Erdalkalimetalloxiden. Gemäß der vorliegenden technischen Lehre ist die Base bevorzugt ausgewählt aus der Gruppe bestehend aus Alkalimetallphosphaten, Alkalimetallcarbonaten und Erdalkalimetalloxiden.

Gemäß der vorliegenden technischen Lehre ist das Alkalimetall-Gegenion ausgewählt aus der Gruppe bestehend aus Natrium, Kalium und Lithium. Die bevorzugten Alkalimetall-Gegenionen sind Natrium oder Lithium. Ganz besonders bevorzugt ist, dass Natrium das Alkalimetall-Gegenion ist. Gemäß der vorliegenden technischen Lehre ist das Erdalkalimetall-Gegenion ausgewählt aus der Gruppe bestehend aus Magnesium, Calcium, Strontium und Barium. Die bevorzugten Erdalkalimetall-Gegenionen sind Magnesium, Calcium oder Strontium. Ganz besonders bevorzugt ist, dass das Erdalkalimetall-Gegenion Magnesium oder Calcium ist. Insbesondere bevorzugt ist, dass das Erdalkalimetall-Gegenion Calcium ist.

Gemäß der vorliegenden technischen Lehre ist die Base insbesondere bevorzugt ausgewählt aus der Gruppe bestehend aus Kaliumphosphat, Natriumcarbonat und Calciumoxid, wobei Kaliumphosphat und/oder Calciumoxid weiter bevorzugt sind.

*Hypohalitquelle*

Wie zuvor beschrieben, stellt die Bildung von Monochlor- oder Monobromamin den ersten Schritt des Reaktionsmechanismus der Berthelot-Reaktion dar und wird in der Regel in Anwesenheit von Hypochlorit oder Hypobromit erreicht. Hypochlorit oder Hypobromit werden dabei *in situ* aus festen Organochlor- oder Organobromverbindungen gebildet, die bevorzugt eine quantitative Hydrolyse zu den entsprechenden Hypohaliden eingehen. Die erfindungsgemäße Hypohalidquelle ist Dichlorisocyanursäure, Trichlorcyanursäure und/oder deren Salze. Bevorzugt als Hypohalitquelle ist dabei Dichlorisocyanursäure und deren Alkali- oder Erdalkalimetallsalze, wobei ein Alkalimetallsalz, wie ein Natrium oder Kaliumsalz der Dichlorisocyanursäure besonders bevorzugt ist.

*Phenolverbindung*

Die Phenolverbindung gemäß der vorliegenden technischen Lehre hat eine unsubstituierte para-Position, die zudem sterisch ungehindert ist oder es handelt sich um Phenole mit einer Halogen-substituierten para-Position.

Die Phenolverbindung gemäß der vorliegenden technischen Lehre ist ein ortho- und/oder meta-substituiertes Phenol gemäß der folgenden Struktur:

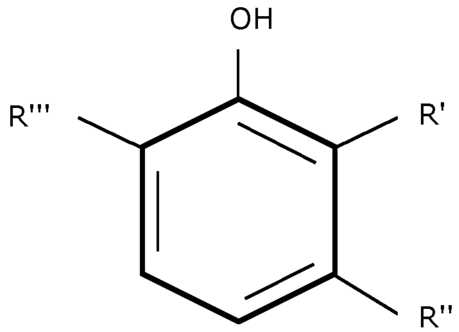

*Abb. 3 Substitutionsmuster eines Phenols gemäß der vorliegenden technischen Lehre*

wobei die Substituenten R', R" und R‴ unabhängig voneinander ausgewählt sind aus H, Alkyl, Aryl, $CO_2H$, $CO_2Li$, $CO_2Na$, $CO_2K$, CO, C(O)N$Alkyl_2$, C(O)$NH_2$, C(S)OAlkyl, C(O)SAlkyl, $CO_2$Alkyl, OAlkyl, SAlkyl, $CH(OAlkyl)_2$, CH(OAlkyl)(SAlkyl), $CH(SAlkyl)_2$, F, Cl, Br, I, OH und SH. R' und R" können an der in Abbildung 3 gezeigten Struktur einen kondensierten aromatischen Ring bilden, sodass ein bi- oder trizyklisches Ringsystem entsteht. Bevorzugt ist, dass die Substituenten R', R" und R‴ unabhängig voneinander ausgewählt sind aus H, Alkyl, Aryl, $CO_2H$, OAlkyl und Cl.

Alkyl ist eine Kohlenwasserstoffgruppe der allgemeinen Formel $C_nH_{2n+1}$, wobei "n" der Anzahl der Kohlenstoffatome entspricht und einen ganzzahligen Wert im Bereich von 1 bis 18, 1 bis 10, 1 bis 5 oder 1 bis 3 annehmen kann. Es ist bevorzugt, dass n 1, 2 oder 3 entspricht. Ganz besonders bevorzugt ist, dass n 1 oder 3 entspricht. Falls n einem Wert von 3 und mehr entspricht, kann die Alkylgruppe linear oder verzweigt sein.

Aryl eine aromatische Kohlenstoffgruppe mit 5 bis 10 Kohlenstoffen. Bevorzugt sind aromatische $C_5$ oder $C_6$ Ringe, welche $C_1$-$C_6$ Alkyl-, Carboxy- und/oder Hydroxygruppen als Substituenten aufweisen können. Besonders bevorzugt ist, dass Aryl ein aromatischer $C_6$ Ring ist, der mit $C_1$-$C_6$ Alkyl-, Carboxy- und/oder Hydroxygruppen substituiert sein kann.

Die Phenolverbindung kann ausgewählt sein aus der Gruppe bestehend aus Phenol, Thymol, Salicylsäure und/oder dessen Salze, α-Naphthol, Guajacol, o-Phenylphenol, o-Chlorphenol, 2-Methyl-5-hydroxychinolin und m-Kresol. Bevorzugte Phenolverbindungen sind Thymol und Natriumsalicylat. Besonders bevorzugt ist Natriumsalicylat als Phenolverbindung.

***Zusammensetzung des Reagenzes***

*Ausführungsformen*

Gemäß der vorliegenden technischen Lehre kann das feststoffliche Reagenz Calciumoxid, Natriumsalicylat, Dichlorisocyanursäure und Nitroprussidnatrium umfassen.

Gemäß der vorliegenden technischen Lehre kann das feststoffliche Reagenz Natriumcarbonat, Natriumsalicylat, Dichlorisocyanursäure und Nitroprussidnatrium umfassen.

Gemäß der vorliegenden technischen Lehre kann das feststoffliche Reagenz Lithiumcarbonat, Natriumsalicylat, Dichlorisocyanursäure und Nitroprussidnatrium umfassen.

Gemäß der vorliegenden technischen Lehre kann das feststoffliche Reagenz Kaliumphosphat, Natriumsalicylat, Dichlorisocyanursäure und Nitroprussidnatrium umfassen.

Gemäß der vorliegenden technischen Lehre kann das feststoffliche Reagenz Calciumoxid, Natriumsalicylat, Dichlorisocyanursäure, Nitroprussidnatrium und Natriumchlorid umfassen.

Gemäß der vorliegenden technischen Lehre kann das feststoffliche Reagenz Natriumcarbonat, Natriumsalicylat, Dichlorisocyanursäure, Nitroprussidnatrium und Natriumchlorid umfassen.

Gemäß der vorliegenden technischen Lehre kann das feststoffliche Reagenz Lithiumcarbonat, Natriumsalicylat, Dichlorisocyanursäure, Nitroprussidnatrium und Natriumchlorid umfassen.

Gemäß der vorliegenden technischen Lehre kann das feststoffliche Reagenz Kaliumphosphat, Natriumsalicylat, Dichlorisocyanursäure, Nitroprussidnatrium und Natriumchlorid umfassen.

Gemäß der vorliegenden technischen Lehre kann das feststoffliche Reagenz Calciumoxid, Natriumsalicylat, Dichlorisocyanursäure, Nitroprussidnatrium, Zitronensäure und Natriumchlorid umfassen.

Gemäß der vorliegenden technischen Lehre kann das feststoffliche Reagenz Natriumcarbonat, Natriumsalicylat, Dichlorisocyanursäure, Nitroprussidnatrium, Zitronensäure und Natriumchlorid umfassen.

Gemäß der vorliegenden technischen Lehre kann das feststoffliche Reagenz Lithiumcarbonat, Natriumsalicylat,

Dichlorisocyanursäure, Nitroprussidnatrium, Zitronensäure und Natriumchlorid umfassen.

Gemäß der vorliegenden technischen Lehre kann das feststoffliche Reagenz Kaliumphosphat, Natriumsalicylat, Dichlorisocyanursäure, Nitroprussidnatrium, Zitronensäure und Natriumchlorid umfassen.

Gemäß der vorliegenden technischen Lehre kann das feststoffliche Reagenz Calciumoxid, Natriumsalicylat, Natriumdichlorisocyanurat und Nitroprussidnatrium umfassen.

Gemäß der vorliegenden technischen Lehre kann das feststoffliche Reagenz Natriumcarbonat, Natriumsalicylat, Natriumdichlorisocyanurat und Nitroprussidnatrium umfassen.

Gemäß der vorliegenden technischen Lehre kann das feststoffliche Reagenz Lithiumcarbonat, Natriumsalicylat, Natriumdichlorisocyanurat und Nitroprussidnatrium umfassen.

Gemäß der vorliegenden technischen Lehre kann das feststoffliche Reagenz Kaliumphosphat, Natriumsalicylat, Natriumdichlorisocyanurat und Nitroprussidnatrium umfassen.

Gemäß der vorliegenden technischen Lehre kann das feststoffliche Reagenz Calciumoxid, Natriumsalicylat, Natriumdichlorisocyanurat, Nitroprussidnatrium und Natriumchlorid umfassen.

Gemäß der vorliegenden technischen Lehre kann das feststoffliche Reagenz Natriumcarbonat, Natriumsalicylat, Natriumdichlorisocyanurat, Nitroprussidnatrium und Natriumchlorid umfassen.

Gemäß der vorliegenden technischen Lehre kann das feststoffliche Reagenz Lithiumcarbonat, Natriumsalicylat, Natriumdichlorisocyanurat, Nitroprussidnatrium und Natriumchlorid umfassen.

Gemäß der vorliegenden technischen Lehre kann das feststoffliche Reagenz Kaliumphosphat, Natriumsalicylat, Natriumdichlorisocyanurat, Nitroprussidnatrium und Natriumchlorid umfassen.

Gemäß der vorliegenden technischen Lehre kann das feststoffliche Reagenz Calciumoxid, Natriumsalicylat, Natriumdichlorisocyanurat, Nitroprussidnatrium, Zitronensäure und Natriumchlorid umfassen.

Gemäß der vorliegenden technischen Lehre kann das feststoffliche Reagenz Natriumcarbonat, Natriumsalicylat, Natriumdichlorisocyanurat, Nitroprussidnatrium, Zitronensäure und Natriumchlorid umfassen.

Gemäß der vorliegenden technischen Lehre kann das feststoffliche Reagenz Lithiumcarbonat, Natriumsalicylat, Natriumdichlorisocyanurat, Nitroprussidnatrium, Zitronensäure und Natriumchlorid umfassen.

Gemäß der vorliegenden technischen Lehre kann das feststoffliche Reagenz Kaliumphosphat, Natriumsalicylat, Natriumdichlorisocyanurat, Nitroprussidnatrium, Zitronensäure und Natriumchlorid umfassen.

*Verhältnis der Komponenten*

In der Berthelot-Reaktion besteht ein komplexer Zusammenhang zwischen der Konzentration der Reaktanden und den Reaktionszwischenprodukten, sodass die Wahl optimaler Konzentrationen erschwert wird.

Gemäß der vorliegenden technischen Lehre kann das feststoffliche Reagenz bis zu 2,5 % oder bis zu 5 % des Katalysators umfassen (in Gewichtsprozent in Bezug auf das Gesamtgewicht des getrockneten Reagenzes angegeben).

Gemäß der vorliegenden technischen Lehre kann das feststoffliche Reagenz mindestens 1 %, mindestens 1,5 % oder mindestens 2 % oder von 1 % bis 94 %, von 1 % bis 70 %, von 1,5 % bis 55 %, von 5 % bis 40 % oder von 10 bis 30 % der Base umfassen (in Gewichtsprozent in Bezug auf das Gesamtgewicht des getrockneten Reagenzes angegeben). Ebenfalls denkbar ist, dass das feststoffliche Reagenz 1 %, 1,5 %, 5 %, 10 %, 30 %, 40 %, 55 % oder 70 % bis 94 % der Base umfasst (in Gewichtsprozent in Bezug auf das Gesamtgewicht des getrockneten Reagenzes angegeben).

Gemäß der vorliegenden technischen Lehre ist bevorzugt, dass das feststoffliche Reagenz die Base in einer Menge umfasst, die gewährleistet, dass bei Zugabe des Reagenzes zur wässrigen (zu analysierenden/bestimmenden) Lösung, die Löslichkeit der Base in Wasser unter Normalbedingungen (Stoffkonstante) nicht überschritten wird.

Gemäß der vorliegenden technischen Lehre kann das feststoffliche Reagenz bis zu 2,5 % oder bis zu 5 % der Hypohalitquelle umfassen (in Gewichtsprozent in Bezug auf das Gesamtgewicht des getrockneten Reagenzes angegeben).

Gemäß der vorliegenden technischen Lehre kann das feststoffliche Reagenz von 1 % bis 94 %, von 5 % bis 70 %, von 5 % bis 50 %, von 5 % bis 40 %, von 10 % bis 30 % oder von 10 % bis 25 % des Phenols umfassen (in Gewichtsprozent in Bezug auf das Gesamtgewicht des getrockneten Reagenzes angegeben). Das feststoffliche Reagenz umfasst bevorzugt 10 bis 25 Gewichtsprozent des Phenols in Bezug auf das Gesamtgewicht des getrockneten Reagenzes.

Gemäß der vorliegenden technischen Lehre kann das feststoffliche Reagenz einen beliebigen Anteil an Additiven umfassen, der nur über den Anteil der restlichen Bestandteile bestimmt ist über die folgende Formel:

*Gew.* % (*Additive*) = 100% - *Gew.* % (*Katalysator*) - *Gew.* % (*Base*) - *Gew.* % (*Hypohalitquelle*) - *Gew.* % (*Phenolverbindung*)

Die Komponenten des erfindungsgemäßen Reagenzes sind, wenn hier nicht anderweitig angegeben, in Gewichtsprozent in Bezug auf das Gesamtgewicht des getrockneten Reagenzes angegeben.

Die Komponenten (Katalysator:Base:Hypohalitquelle:Phenolverbindung:Additive - normiert auf den Anteil der Base) können in einem der folgenden Verhältnisse vorliegen:

bis 0,40:1: bis 0,34: bis 9,27: bis 30,00;

bis 0,50:1: bis 0,43: bis 11,62: bis 37,61;

bis 0,01:1: bis 0,01: bis 0,23: bis 0,75; oder

bis 0,05:1: bis 0,04: bis 1,16: bis 3,74.

*Additive*

Gemäß der vorliegenden technischen Lehre kann jede Ausgestaltungsform Additive enthalten.

Gemäß der vorliegenden technischen Lehre kann das Reagenz von 0,01 bis zu 0,5, 0,1, 0,05 oder 0,02 Gew.-%, bis zu 0,1 Gew.-%, bis zu 0,05 Gew.-% oder bis zu 0,02 Gew.-% Wasser enthalten. Es ist bevorzugt, dass das erfindungsgemäße Reagenz frei von Wasser ist.

Gemäß der vorliegenden technischen Lehre sind die Additive ausgewählt aus der Gruppe bestehend aus einem oder mehreren Chelatbildnern, Füllstoffe und Kombinationen derselben.

Der eine oder die mehreren Chelatbildner, die gemäß der vorliegenden technischen Lehre Anwendung finden können, sind ausgewählt aus der Gruppe bestehend aus Alkali- und Erdalkalimetallsalzen von Ethylendiamintetraessigsäure (EDTA), Zitronensäure, Weinsäure und Kombinationen derselben. Bevorzugte Chelatbildner sind Alkalimetallsalze von EDTA, Zitronensäure und/oder Weinsäure.

Gemäß der vorliegenden technischen Lehre ist das Alkalimetall-Gegenion ausgewählt aus der Gruppe bestehend aus Natrium, Kalium und Lithium. Die bevorzugten Alkalimetall-Gegenionen sind Natrium oder Kalium. Ganz besonders bevorzugt ist, dass Natrium das Alkalimetall-Gegenion ist. Gemäß der vorliegenden technischen Lehre ist das Erdalkalimetall-Gegenion ausgewählt aus der Gruppe bestehend aus Magnesium, Calcium, Strontium und Barium. Die bevorzugten Erdalkalimetall-Gegenionen sind Magnesium, Calcium oder Strontium. Ganz besonders bevorzugt ist, dass das Erdalkalimetall-Gegenion Magnesium oder Calcium ist. Insbesondere bevorzugt ist, dass das Erdalkalimetall-Gegenion Calcium ist.

Besonders bevorzugte Chelatbildner sind Trinatriumcitrat und/oder Dinatriumtartrat.

Füllstoffe, die gemäß der vorliegenden technischen Lehre Anwendung finden können, sind ausgewählt aus Natriumchlorid, Mannit, Natriumsulfat und Calciumchlorid.

Das erfindungsgemäße feststoffliche Reagenz kann von 0,01 bis zu 0,5 Gew.-% Wasser, in Bezug auf das Gesamtgewicht des Reagenzes, enthalten oder kann frei von Wasser sein.

Das erfindungsgemäße feststoffliche Reagenz kann pulverförmig oder als Pulverkissen vorliegen. Dabei ist bevorzugt, dass das Pulverkissen 600 mg oder weniger des erfindungsgemäßen feststofflichen Reagenzes und insbesondere 13 bis 17 mg, 26 bis 32 mg, 52 bis 64 mg, 78 bis 96 mg, 104 bis 128 mg, 130 bis 160 mg oder 156 bis 192 mg des erfindungsgemäßen feststofflichen Reagenzes umfasst.

Weiterhin kann das feststoffliche Reagenz in Tablettenform vorliegen.

**Messmethoden**

***Qualitativer Nachweis***

Das Verfahren zum qualitativen Nachweis von Ammonium-Ionen in wässriger Lösung umfasst die folgenden Schritte:

a) Bereitstellen einer Probelösung;

b) Zugabe des feststofflichen Reagenzes gemäß der vorliegenden technischen Lehre, wobei die Menge des zugegebenen Reagenzes vom Volumen der Probelösung abhängig ist;

c) Vollständiges oder teilweises Auflösen des zugegebenen feststofflichen Reagenzes;

d) Inkubation unter Normalbedingungen für 30 Minuten oder bis eine Verfärbung der Reaktionslösung eingetreten ist.

Hierbei sei anzumerken, dass bei der Berthelot-Reaktion zwar ein blauer Farbstoff entsteht, die objektiv-visuell

erfasste Verfärbung allerdings von den Komponenten des Reagenzes abhängig ist (Verfärbung bedeutet hierbei die Bildung der Mischfarbe zwischen der Farbe der Lösung und des sich bildenden blauen Farbstoffs). So ergibt sich z. B. optisch eine Grünfärbung der Reaktionslösung, sofern Natriumhexacyanoferrat als Katalysator verwendet wird durch Bildung einer Mischfarbe, denn Natriumhexacyanidoferrat ist als Decahydrat ein gelber Feststoff.

***Quantitative Bestimmung***

Das Verfahren zur quantitativen Bestimmung von Ammonium-Ionen in wässriger Lösung umfasst die folgenden Schritte:

a) Bereitstellen einer Probelösung;

b) Pipettieren der Probelösung in eine Küvette;

c) Zugabe des feststofflichen Reagenzes gemäß der vorliegenden technischen Lehre, wobei die Menge des zugegebenen Reagenzes vom Volumen der Probelösung abhängig ist;

d) Vollständiges oder teilweises Auflösen des zugegebenen feststofflichen Reagenzes;

e) Inkubation unter Normalbedingungen für 30 min;

f) Photometrische Extinktionsbestimmung der Lösung bei einer Wellenlänge von 680 nm.

In den beschriebenen Verfahren (quantitativer Nachweis und qualitative Bestimmung) werden für 1 mL Probelösung 10 bis 40 mg und bevorzugterweise 15 bis 30 mg des feststofflichen Reagenzes zugegeben.

**Experimente**

Herkömmliche Mitbewerberprodukte, welche sich die Indophenolblau-Methode (Berthelot-Reaktion) zu Nutze machen, verwenden wenigstens zwei unterschiedliche Reagenzien. So beinhalten die Reagenzien der Firma HACH ein "salicylate reagent" und ein "cyanuric acid reagent" (Tabelle 1, Eintrag 1), bei welchem die oben aufgeführten Reagenzien auf zwei sog. Pulverkissen aufgeteilt sind. Beim Ammoniumküvettentest LCK 304 sind Natriumsalicylate und Dichlorisocyanursäure ebenfalls durch das Reagenz in der Rundküvette und dem Dosicap getrennt (Tabelle 1, Eintrag 2). Auch ein drittes Produkt der Firma HACH "AmVer" trennt die einzelnen Komponenten auf zwei Reagenzien auf (Tabelle 1, Eintrag 3). Bei einem Ammonium-Test der Firma Merck, welcher mittels Farbkarte ausgewertet werden kann, sind die einzelnen Komponenten der Reaktion gar auf drei unterschiedliche Reagenzien aufgeteilt (Tabelle 1, Eintrag 4). Weitere Beispiele für Mehrkomponentensysteme von Merck, Lovibond und MACHEREY-NAGEL sind aufgeführt (Tabelle 1, Einträge 5 bis 7).

*Tabelle 1 Gegenüberstellung der kommerziell erhältlichen (üblichen) Systeme und dem erfindungsgemäßen Reagenz*

| Eintrag | Testkit | Phenolverbindung | Base | Katalysator | Hypohalitquelle |
|---|---|---|---|---|---|
| 1 | Salicylate reagent 2653299 | Natriumsalicylat | | Nitroprussidnatrium | |
| | Cyanurate reagent 2653199 | | Dinatriumtartrat LiOH | | Dichlorisocyanursäure |
| 2 | LCK 304 Rundküvette | Natriumsalicylat | Trinatriumcitrat NaOH | | |
| | LCK 304 Dosicap | | | Nitroprussidnatrium | Dichlorisocyanursäure |
| 3 | HACH AmVer 2604545 Salicylate reagent | Natriumsalicylate | Trinatriumcitrat Dinatriumtartrat | Nitroprussidnatrium | |
| | HACH AmVer 2604545 Cyanurate reagent | | Trinatriumcitrat Dinatriumtartrat LiOH | | Dichlorisocyanursäure |

(fortgesetzt)

| Eintrag | Testkit | Phenolverbindung | Base | Katalysator | Hypohalitquelle |
|---|---|---|---|---|---|
| **4** | MQuant 114428 Ammoniumtest (Merck) Reagent 1 | | NaOH | | |
| | MQuant 114428 Ammoniumtest (Merck) Reagent 2 | | | | Dichlorisocyanursäure |
| | MQuant 114428 Ammoniumtest (Merck) Reagent 3 | Thymol | | Nitroprussidnatrium | |
| **5** | MACHEREY NAGEL 985003 Reagenz 1 | Natriumsalicylat | Trinatriumcitrat NaOH | | |
| | MACHEREY NAGEL 985003 Reagenz 2 (Nanofix) | | | Nitroprussidnatrium | Dichlorisocyanursäure |
| **6** | Ammonium mit Tablette No.1 Lovibond 00512581 | Salicylsäure | | Nitroprussidnatrium | |
| | Ammonium mit Tablette No.2 Lovibond 00512591 | | LiOH | | Dichlorisocyanursäure |
| **7** | Ammonium mit Vario Pulverpäckchen Salicylate Reagent | Natriumsalicylat | | Nitroprussidnatrium | |
| | Ammonium mit Vario Pulverpäckchen Cyanurat Reagent | | LiOH | | Dichlorisocyanursäure |
| **8a** | Powder Pillow Ammonium mit CaO | Natriumsalicylat | CaO | Nitroprussidnatrium | Dichlorisocyanursäure |
| **8b** | Powder Pillow Ammonium mit K3PO4 | Natriumsalicylat | $K_3PO_4$ | Nitroprussidnatrium | Dichlorisocyanursäure |
| **8c** | Powder Pillow Ammonium mit Na2CO3 | Natriumsalicylat | $Na_2CO_3$ | Nitroprussidnatrium | Dichlorisocyanursäure |

Der Einsatz von CaO hat sich als vorteilhaft herausgestellt. Die Pulvermischung liefert gute Messergebnisse im Bereich von 0,05 - 0,50 mg/L Ammonium (*Eintrag 8a*). Der Lagerversuch in Form von Pulverkissen zeigte eine Lagerfähigkeit von 2 Jahren. Auch Trikaliumphosphat liefert als Base gute Messergebnisse im Bereich von 0,05 - 0,50 mg/L Ammonium (*Eintrag 8b*).

***Proben***

Verwendete Chemikalien:

Nitroprussidnatrium (CAS: 13755-38-9; von Merck, Bestellnummer: 1065410500)

Natriumsalicylat (CAS: 54-21-7; von Bernd Kraft, Artikel-Nr.: BK17418.3600)

NaCl (CAS: 7647-14-5; von Merck, Bestellnummer: 106404)

Natriumdichlorisocyanurat (CAS: 52671-45-1; von Acros, Artikelnummer: 11300290)

CaO (CAS: 1305-78-8; von Acros, Katalog-Nr.: AC422830010)

$Na_2CO_3$ (CAS: 497-19-8, von Merck, Katalog-Nr.: 106392)

$Li_2CO_3$ (CAS: 554-13-2, von Sigma-Aldrich, Katalog-Nr.: 255823)

$K_3PO_4 \cdot 3H_2O$ (CAS: 22763-03-7, von Merck, Katalog-Nr.: 1.05102)

Die nachfolgenden Reagenzien wurden getestet:

**Reagenz 1 (APP019):**

| | |
|---|---|
| 11,6 g | Nitroprussidnatrium |
| 272 g | Natriumsalicylat |
| 880 g | NaCl |
| 10 g | Natriumdichlorisocyanurat |
| 29,33 g | CaO |

**Reagenz 2 (APP020):**

| | |
|---|---|
| 11,6 g | Nitroprussidnatrium |
| 272 g | Natriumsalicylat |
| 880 g | NaCl |
| 10 g | Natriumdichlorisocyanurat |
| 23,4 g | CaO |

**Reagenz 3 (APP011):**

| | |
|---|---|
| 11,6 g | Nitroprussidnatrium |
| 272 g | Natriumsalicylat |
| 880 g | NaCl |
| 10 g | Natriumdichlorisocyanurat |
| 1175 g | $Na_2CO_3$ |

**Reagenz 4 (APP010):**

| | |
|---|---|
| 11,6 g | Nitroprussidnatrium |
| 272 g | Natriumsalicylat |
| 880 g | NaCl |
| 10 g | Natriumdichlorisocyanurat |
| 235 g | $Na_2CO_3$ |

**Reagenz 5 (APP009):**

| | |
|---|---|
| 11,6 g | Nitroprussidnatrium |
| 272 g | Natriumsalicylat |
| 880 g | NaCl |
| 10 g | Natriumdichlorisocyanurat |
| 1173,6 g | $Li_2CO_3$ |

**Reagenz 6 (APP008):**

| | |
|---|---|
| 11,6 g | Nitroprussidnatrium |
| 272 g | Natriumsalicylat |
| 880 g | NaCl |
| 10 g | Natriumdichlorisocyanurat |
| 235 g | $Li_2CO_3$ |

**Reagenz 7 (APP006):**

| | |
|---|---|
| 11,6 g | Nitroprussidnatrium |
| 272 g | Natriumsalicylat |
| 880 g | NaCl |
| 10 g | Natriumdichlorisocyanurat |
| 1175 g | $K_3PO_4 \cdot 3H_2O$ |

***Kalibrierfunktionen***

Für die Reagenzien wurden Kalibrierreihen mit 5 bzw. 10 Standardlösungen äquidistanter Konzentrationen aufgenommen. Die Testdurchführung ist dabei wie folgt:

1) 1 gr. Messlöffel (ca. 130 - 160 mg) des erfindungsgemäßen Reagenzes zu 5 mL Probelösung in eine 14 mm Rundküvette geben.

2) Rundküvette mit Schraubdeckel verschließen und schütteln.

3) 30 min unter Normalbedingungen inkubieren.

4) Rundküvette von außen säubern.

5) Die Extinktion bei 680 nm im VIS II-Spektralphotometer (MACHEREY-NAGEL) bestimmen.

6) Die Kalibrierfunktion über die erhaltenen Wertepaare erstellen.

Figuren 1, 4, 7, 8 und 9 zeigen derartige Kalibierreihen beispielhalt an Hand der Kalibrierreihen, die für Reagenz 1, 2, 5, 6 und 7 aufgenommen wurden.

***Verfahrenskenndaten***

Für Reagenz 2 wurden die nachfolgenden Verfahrenskenndaten aufgenommen:

*Tabelle 2 Verfahrenskenndaten für Reagenz 2.*

| **Verfahrenskenndaten** | |
|---|---|
| **Standardabweichung** | 0,005 mE |
| **Verfahrensstandardabweichung** | 0,004 mg/L |
| **Steigung** | 1287,68 mE/(mg/L) |
| **Ordinatenabschnitt** | 35,2 mE |
| **Nachweisgrenze (Schätzwert)** | 0,015 mg/L |
| **Bestimmungsgrenze (Schätzwert)** | 0,046 mg/L |
| **Verfahrensvariationskoeffizient Vx,o** | 0,96 % |

Bei der Einheit [mE] handelt es sich um die Milliextinktion gemäß:

$$E = \log (I_0/I_1)$$

wobei $I_0$ der Intensität des einfallenden (eingestrahlten) Lichtes und $I_1$ der Intensität des transmittierten Lichtes entspricht.

***Langzeitlagerversuch***

Ein anschließender Lagerversuch in einem handelsüblichen Trockenschrank der Firma Memmert (Universalschrank UF160plus) bei 50 °C erlaubt eine Einschätzung und Extrapolation der Lagerfähigkeit der Pulvermischung (siehe Figuren 2, 5 und 6). Der Einfluss der Stressbedingungen bei der Lagerung wurde dadurch quantifiziert, dass in regelmäßigen Abständen das erfindungsgemäße Verfahren zur quantitativen Bestimmung von Ammonium-Ionen in wässriger Lösung für eine Probe bekannter Ammonium-Ionenkonzentration (Sollwert = 0,5 mg/L, siehe gestrichelte Linie) angewandt wird. Hierzu wurde die nach obigen Angaben erhaltene Kalibrierfunktion verwendet. Die Extinktionsmessung erfolgte bei 680 nm im VIS II- Spektralphotometer (MACHEREY-NAGEL). Aus diesem Lagerversuch lässt sich die Haltbarkeit der Pulvermischung zuverlässig für etwa 3 Jahren extrapolieren.

Die Reagenzien liefern gute Ergebnisse in Kalibration und im Lagerversuch. Eine Abweichung, die über die Fehlertoleranz (Fehlertoleranz von 10 %, bei einem Sollwert von 0,5 mg/L entspricht dies ±0,05 mg/L, siehe gepunktete Linien) hinausgeht, wurde für das erfindungsgemäße Reagenz nicht beobachtet.

***Einfluss der Reagenzmenge***

Die Versuchsreihe, deren Ergebnisse in Tabelle 3 zusammengefasst sind, wurde an Hand der nachfolgenden Schritte durchgeführt:

a) Vorlegen des feststofflichen Reagenzes 2 in einer Rundküvette mit 16 mm Außendurchmesser;

b) Hinzugeben vom 5 mL an Probelösung mit einer Ammoniumsollkonzentration von 0,5 mg/L;

b) Verschließen und Schütteln der Rundküvette;

c) Inkubation unter Normalbedingungen für 30 min;

d) Photometrische Extinktionsbestimmung der Lösung bei einer Wellenlänge von 680 nm.

*Tabelle 3 Abhängigkeit des Messergebnisses bezogen auf die Reagenzmenge an Reagenz 2*

| Bemerkung | Reagenzmenge | Reagenzmenge pro mL Probevolumen | Gemessene Konzentration | Abweichung vom Sollwert |
|---|---|---|---|---|
| | 12,5 mg | 2,5 mg/mL | 0,05 mg/mL | -90 % |
| | 25,0 mg | 5,0 mg/mL | 0,34 mg/mL | -32 % |
| | 50,0 mg | 10,0 mg/mL | 0,45 mg/mL | -10 % |
| | 75,0 mg | 15,0 mg/mL | 0,49 mg/mL | -2 % |
| | 100,0 mg | 20,0 mg/mL | 0,50 mg/mL | 0 % |
| | 150,0 mg | 30,0 mg/mL | 0,52 mg/mL | 4 % |
| | 200,0 mg | 40,0 mg/mL | 0,53 mg/mL | 6 % |
| trüb | 300,0 mg | 60,0 mg/mL | 0,63 mg/mL | 26 % |
| trüb | 400,0 mg | 80,0 mg/mL | 0,68 mg/mL | 36 % |
| trüb | 500,0 mg | 100,0 mg/mL | 0,67 mg/mL | 34 % |

Tabelle 3 zeigt die Abhängigkeit des Messergebnisses bezogen auf die Reagenzmenge. Reagenzmengen von 10 bis 40 mg pro Milliliter an Probenvolumen liefern besonders hohe Messpräzision.

***Kurzzeitlagerstabilität***

Um die Lagerstabilität des erfindungsgemäßen feststofflichen Reagenzes und die der Mischungen mit Hydroxiden als Base zu untersuchen, wurden die in Tabelle 4 zusammengefassten Zusammensetzungen vorbereitet. Hierzu wurden in allen Fällen Katalysator, Base, Hypohalitquelle und Phenolverbindung gemischt und durch Mörsern homogenisiert. Als Katalysator, Hypohalitquelle und Phenolverbindung wurden in allen Beispielen Nitroprussidnatrium, Dichlorisocyanursäure (DCI) und Natriumsalicylat verwendet, wobei Natriumchlorid als Füllstoff eingesetzt wurde (Natriumchlorid macht die restlichen Gew.% in Bezug auf das Gesamtgewicht der getrockneten Zusammensetzung aus). Diese Zusammensetzungen wurden 2 Tage unter Normalbedingungen gelagert, wobei diese zunächst stündlich, dann täglich in Bezug auf äußerliche Veränderungen untersucht wurden.

Auf diesem Wege konnten die erfindungsgemäßen Basen (CaO, $Na_2CO_3$ und $K_3PO_4$) direkt mit den Hydroxiden verglichen werden, die in kommerziell erhältlichen Mehrkomponentensystemen Anwendung finden. In diesem Zusammenhang wurden hierbei auch der Einfluss der Menge der Base in der Zusammensetzung (in Bezug auf das Gesamtgewicht der getrockneten Zusammensetzung) auf die Lagerstabilität untersucht.

*Tabelle 4 Auf Kurzzeitlagerstabilität getestete Zusammensetzungen*

| # | Base | Anteil in Gew.% | Katalysator | Anteil in Gew.% | Hypohalitquelle | Anteil in Gew.% | Phenolverbindung | Anteil in Gew.% |
|---|---|---|---|---|---|---|---|---|
| **1** | CaO | 5 | NPN | 0,94 | DCI | 0,81 | Natriumsalicylat | 22 |
| **2** | $Na_2CO_3$ | 10 | NPN | 0,89 | DCI | 0,77 | Natriumsalicylat | 21 |
| **3** | $K_3PO_4•3H_2O$ | 10 | NPN | 0,89 | DCI | 0,77 | Natriumsalicylat | 21 |
| **4** | LiOH | 2 | NPN | 0,97 | DCI | 0,84 | Natriumsalicylat | 23 |
| **5** | LiOH | 5 | NPN | 0,94 | DCI | 0,81 | Natriumsalicylat | 22 |
| **6** | LiOH | 10 | NPN | 0,89 | DCI | 0,77 | Natriumsalicylat | 21 |
| **7** | NaOH | 2 | NPN | 0,97 | DCI | 0,84 | Natriumsalicylat | 23 |
| **8** | NaOH | 5 | NPN | 0,94 | DCI | 0,81 | Natriumsalicylat | 22 |
| **9** | NaOH | 10 | NPN | 0,89 | DCI | 0,77 | Natriumsalicylat | 21 |
| **10** | KOH | 2 | NPN | 0,97 | DCI | 0,84 | Natriumsalicylat | 23 |
| **11** | KOH | 5 | NPN | 0,94 | DCI | 0,81 | Natriumsalicylat | 22 |

Bereits nach nur wenigen Stunden konnte eine gelbliche Verfärbung der Zusammensetzungen 7 bis 11 objektiv (visuell) wahrgenommen werden. Zusammensetzungen 4 bis 6 zeigten eine leichte Gelbfärbung nach einem Tag. Für die Zusammensetzungen 4 bis 11 konnte überdies eine Verklumpung beobachtet werden. Diese Effekte nahmen mit zunehmendem Anteil an Hydroxid an der Zusammensetzung zu. Die erfindungsgemäßen Zusammensetzungen blieben hingegen feinpulvrig (kein Verklumpen) und zeigten keinerlei Verfärbungen.

**Patentansprüche**

1. Ein feststoffliches Reagenz zur Bestimmung von Ammonium-Ionen in wässriger Lösung umfassend oder im Wesentlichen bestehend aus einem Gemisch der nachfolgenden Komponenten:

   (i) Katalysator,
   (ii) Base,
   (iii) Hypohalitquelle, und
   (iv) Phenolverbindung,
   wobei der Katalysator Nitroprussidnatrium oder Natriumhexacyanoferrat ist; wobei die Base einen $pK_B$-Wert von -2 bis 4 aufweist mit der Maßgabe, dass die Base kein Hydroxid ist; und
   wobei die Phenolverbindung ein ortho- und/oder meta-substituiertes Phenol gemäß der folgenden Struktur ist:

   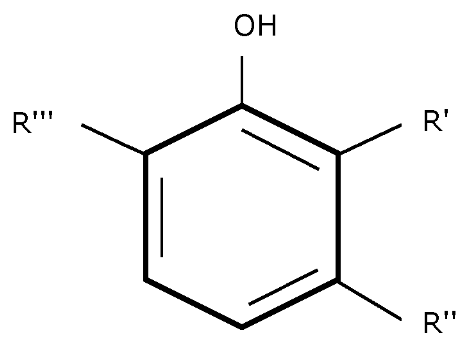

   wobei die Substituenten R', R" und R‴ unabhängig voneinander ausgewählt sind aus der Gruppe bestehend aus H, Alkyl, Aryl, $CO_2H$, $CO_2Li$, $CO_2Na$, $CO_2K$, CO, $C(O)NAlkyl_2$, $C(O)NH_2$, C(S)OAlkyl, C(O)SAlkyl, $CO_2Alkyl$, OAlkyl, SAlkyl, $CH(OAlkyl)_2$, CH(OAlkyl)(SAlkyl), $CH(SAlkyl)_2$, F, Cl, Br, I, OH und SH;
   wobei Alkyl eine Kohlenwasserstoffgruppe der allgemeinen Formel $C_nH_{2n+1}$ ist, wobei "n" der Anzahl der Kohlenstoffatome entspricht und ein ganzzahliger Wert im Bereich von 1 bis 18 ist; und
   Aryl eine aromatische Kohlenstoffgruppe mit 5 bis 10 Kohlenstoffen ist.

2. Feststoffliches Reagenz gemäß Anspruch 1, **dadurch gekennzeichnet, dass** der Katalysator Nitroprussidnatrium ist.

3. Feststoffliches Reagenz gemäß einem oder mehreren der vorangehenden Ansprüche, **dadurch gekennzeichnet, dass** die Base ausgewählt ist aus der Gruppe bestehend aus Alkali- und/oder Erdalkalimetallphosphaten, Alkali- und/oder Erdalkalimetallcarbonaten und Alkali- und/oder Erdalkalimetalloxiden.

4. Feststoffliches Reagenz gemäß einem oder mehreren der vorangehenden Ansprüche, **dadurch gekennzeichnet, dass** die Base ausgewählt ist aus der Gruppe bestehend aus Alkalimetallphosphaten, Alkalimetallcarbonaten oder Erdalkalimetalloxiden.

5. Feststoffliches Reagenz gemäß einem oder mehreren der vorangehenden Ansprüche, **dadurch gekennzeichnet, dass** die Hypohalitquelle ausgewählt ist aus der Gruppe bestehend aus Dichlorisocyanursäure, Trichlorcyanursäure und/oder deren Salze.

6. Feststoffliches Reagenz gemäß einem oder mehreren der vorangehenden Ansprüche, **dadurch gekennzeichnet, dass** die Phenolverbindung ausgewählt ist aus der Gruppe bestehend aus Phenol, Thymol, Salicylsäure und/oder deren Salze, $\alpha$-Naphthol, Guajacol, o-Phenylphenol, o-Chlorphenol, 2-Methyl-5-hydroxychinolin und m-Kresol.

7. Feststoffliches Reagenz gemäß einem oder mehreren der vorangehende Ansprüche, **dadurch gekennzeichnet, dass** das feststoffliche Reagenz mindestens 2 Gew.% an Base in Bezug auf das Gesamtgewicht des getrockneten Reagenzes umfasst und/oder mindestens 2 Gew.% an Phenolverbindung in Bezug auf das Gesamtgewicht des getrockneten Reagenzes umfasst.

8. Feststoffliches Reagenz gemäß einem oder mehreren der vorangehenden Ansprüche, **dadurch gekennzeichnet, dass** das feststoffliche Reagenz bis zu 1 Gew.% an (i), bis zu 2,5 Gew.% an (ii), bis zu 1 Gew.% an (iii) und bis zu 25 Gew.% an (iv) in Bezug auf das Gesamtgewicht des getrockneten Reagenzes umfasst, wobei der Rest aus Additiven besteht;

   bis zu 1 Gew.% an (i), bis zu 5 Gew.% an (ii), bis zu 1 Gew.% an (iii) und bis zu 25 Gew.% an (iv) in Bezug auf das Gesamtgewicht des getrockneten Reagenzes umfasst, wobei der Rest aus Additiven besteht;
   bis zu 0,5 Gew.% an (i), bis zu 55 Gew.% an (ii), bis zu 0,5 Gew.% an (iii) und bis zu 15 Gew.% an (iv) in Bezug auf das Gesamtgewicht des getrockneten Reagenzes umfasst, wobei der Rest aus Additiven besteht; oder
   bis zu 1 Gew.% an (i), bis zu 20 Gew.% an (ii), bis zu 0,75 Gew.% an (iii) und bis zu 20 Gew.% an (iv) in Bezug auf das Gesamtgewicht des getrockneten Reagenzes umfasst, wobei der Rest aus Additiven besteht.

9. Feststoffliches Reagenz gemäß einem oder mehreren der vorangehenden Ansprüche, **dadurch gekennzeichnet, dass** das Reagenz frei von Wasser ist.

10. Feststoffliches Reagenz gemäß einem oder mehreren der vorangehenden Ansprüche, wobei

   (i) das Reagenz als Pulverkissen vorliegt; oder
   (ii) das Reagenz in Tablettenform vorliegt.

11. Testkit zur Bestimmung von Ammonium-Ionen in wässriger Lösung umfassend das feststoffliche Reagenz gemäß einem oder mehreren der vorangehenden Ansprüche.

12. Verwendung des feststofflichen Reagenzes gemäß einem oder mehreren der Ansprüche 1 bis 10 zum qualitativen Nachweis und/oder der quantitativen Bestimmung von Ammonium-Ionen in wässriger Lösung.

13. Verfahren zum qualitativen Nachweis von Ammonium-Ionen in wässriger Lösung umfassend die folgenden Schritte:

   a) Bereitstellen einer Probelösung;
   b) Zugabe des feststofflichen Reagenzes gemäß einem oder mehreren der Ansprüche 1 bis 10 zur vorgelegten Probelösung oder Vorlegen des feststofflichen Reagenzes und anschließende Zugabe der Probelösung;
   c) Rühren der erhaltenen Lösung;
   d) Inkubation unter Normalbedingungen für 30 min oder bis eine Verfärbung der Lösung eingetreten ist.

14. Verfahren zur quantitativen Bestimmung von Ammonium-Ionen in wässriger Lösung umfassend die folgenden Schritte:

   a) Bereitstellen einer Probelösung;
   b) Pipettieren der Probelösung in eine Küvette;
   c) Zugabe des feststofflichen Reagenzes einem oder mehreren der Ansprüche 1 bis 10 zur Probelösung;
   d) Vollständiges oder teilweises Auflösen des zugegebenen feststofflichen Reagenzes;
   e) Inkubation unter Normalbedingungen für 30 min;
   f) Photometrische Extinktionsbestimmung der Lösung bei einer Wellenlänge von 680 nm.

15. Verfahren gemäß Anspruch 13 oder 14, **dadurch gekennzeichnet, dass** pro Milliliter Probelösung 10 bis 40 mg und bevorzugt 15 bis 30 mg des feststofflichen Reagenzes verwendet werden.

## Claims

1. A solid-state reagent for assaying ammonium ions in aqueous solution, comprising, or essentially consisting of, a mixture of the following components:

(i) a catalyst,
(ii) a base,
(iii) a hypohalite source, and
(iv) a phenol compound,
wherein said catalyst is sodium nitroprusside or sodium hexacyanoferrate,
wherein said base has a $pK_B$ value of from -2 to +4, with the proviso that the base is not a hydroxide, and wherein the phenol compound is an ortho- and/or meta-substituted phenol according to the following structure:

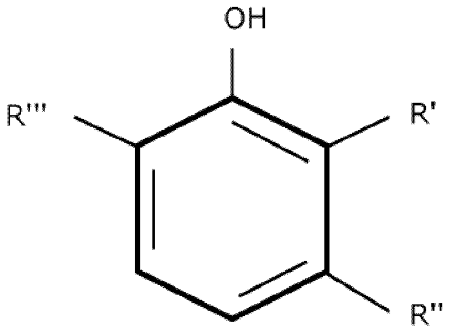

wherein the substituents R', R'' and R''' are independently selected from the group consisting of H, alkyl, aryl, $CO_2H$, $CO_2Li$, $CO_2Na$, $CO_2K$, CO, $C(O)Nalkyl_2$, $C(O)NH_2$, C(S)Oalkyl, C(O)Salkyl, $CO_2alkyl$, Oalkyl, Salkyl, $CH(Oalkyl)_2$, CH(Oalkyl)(Salkyl), $CH(Salkyl)_2$, F, Cl, Br, I, OH and SH;
wherein alkyl is a hydrocarbyl group of general formula $C_nH_{2n+1}$, in which "n" is the number of carbon atoms and is an integer within a range from 1 to 18; and
aryl is an aromatic hydrocarbyl group with 5 to 10 carbon atoms.

2. The solid-state reagent according to claim 1, **characterized in that** said catalyst is sodium nitroprusside.

3. The solid-state reagent according to one or more of the preceding claims, **characterized in that** said base is selected from the group consisting of alkali and/or alkaline earth metal phosphates, alkali and/or alkaline earth metal carbonates, and alkali and/or alkaline earth metal oxides.

4. The solid-state reagent according to one or more of the preceding claims, **characterized in that** said base is selected from the group consisting of alkali metal phosphates, alkali metal carbonates, or alkaline earth metal oxides.

5. The solid-state reagent according to one or more of the preceding claims, **characterized in that** said hypohalite source is selected from the group consisting of dichloroisocyanuric acid, trichlorocyanuric acid, and/or their salts.

6. The solid-state reagent according to one or more of the preceding claims, **characterized in that** said phenol compound is selected from the group consisting of phenol, thymol, salicylic acid and/or its salts, $\alpha$-naphthol, guajacol, o-phenylphenol, o-chlorophenol, 2-methyl-5-hydroxyquinoline, and m-cresol.

7. The solid-state reagent according to one or more of the preceding claims, **characterized in that** said solid-state reagent comprises at least 2% by weight of base, based on the total weight of the dried reagent, and/or at least 2% by weight of phenol compound, based on the total weight of the dried reagent.

8. The solid-state reagent according to one or more of the preceding claims, **characterized in that** said solid-state reagent comprises up to 1% by weight of (i), up to 2.5% by weight of (ii), up to 1% by weight of (iii), and up to 25% by weight of (iv), based on the total weight of the dried reagent, the remainder consisting of additives;

comprises up to 1% by weight of (i), up to 5% by weight of (ii), up to 1% by weight of (iii), and up to 25% by weight of (iv), based on the total weight of the dried reagent, the remainder consisting of additives;
comprises up to 0.5% by weight of (i), up to 55% by weight of (ii), up to 0.5% by weight of (iii), and up to 15% by weight of (iv), based on the total weight of the dried reagent, the remainder consisting of additives; or
comprises up to 1% by weight of (i), up to 20% by weight of (ii), up to 0.75% by weight of (iii), and up to 20% by weight of (iv), based on the total weight of the dried reagent, the remainder consisting of additives.

**9.** The solid-state reagent according to one or more of the preceding claims, **characterized in that** the reagent is free of water.

**10.** The solid-state reagent according to one or more of the preceding claims, wherein

(i) said reagent is in the form of a powder pad; or
(ii) said reagent is in tablet form.

**11.** A test kit for assaying ammonium ions in aqueous solution, comprising the solid-state reagent according to one or more of the preceding claims.

**12.** Use of the solid-state reagent according to one or more of claims 1 to 10 for the qualitative detection and/or the quantitative determination of ammonium ions in aqueous solution.

**13.** A method for the qualitative detection of ammonium ions in aqueous solution comprising the following steps:

a) providing a sample solution;
b) adding the solid-state reagent according to one or more of claims 1 to 10 to the provided sample solution, or providing the solid-state reagent and then adding the sample solution;
c) stirring the solution obtained;
d) incubating under normal conditions for 30 min, or until a discoloration of the solution has occurred.

**14.** A method for the quantitative determination of ammonium ions in aqueous solution comprising the following steps:

a) providing a sample solution;
b) pipetting said sample solution into a cuvette;
c) adding the solid-state reagent according to one or more of claims 1 to 10 to the sample solution;
d) completely or partially dissolving the added solid-state reagent;
e) incubating under normal conditions for 30 min;
f) photometric determination of the absorbance of the solution at a wavelength of 680 nm.

**15.** The method according to claim 13 or 14, **characterized in that** 10 to 40 mg and preferably 15 to 30 mg of the solid-state reagent is used per milliliter of the sample solution.

**Revendications**

**1.** Réactif solide permettant de détecter des ions ammonium en solution aqueuse, comprenant un mélange des composants suivants ou essentiellement constitué de ce mélange :

(i) catalyseur,
(ii) base,
(iii) source d’hypohalite, et
(iv) composé phénolique,
dans lequel le catalyseur est du nitroprussiate de sodium ou de l’hexacyanoferrate de sodium ; dans lequel la base a une valeur de $pK_B$ comprise entre -2 et 4, sous réserve que la base ne soit pas un hydroxyde ; et dans lequel le composé phénolique est un phénol ortho-substitué et/ou méta-substitué correspondant à la structure suivante :

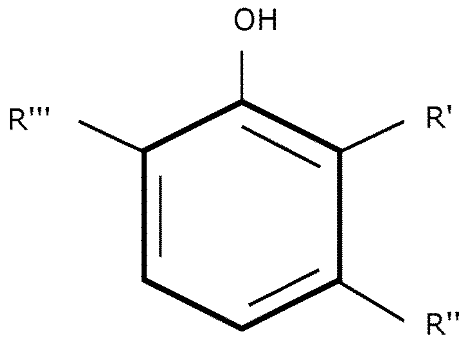

dans laquelle les substituants R’, R" et R’" sont choisis indépendamment les uns des autres dans le groupe constitué de H, alkyle, aryle, $CO_2H$, $CO_2Li$, $CO_2Na$, $CO_2K$, CO, C(O) N-alkyle$_2$, C(O)$NH_2$, C(S)O-alkyle, C(O)S-alkyle, $CO_2$alkyle, O-alkyle, S-alkyle, CH(O-alkyle)$_2$, CH(O-alkyl)(S-alkyle), CH(S-alkyle)$_2$, F, Cl, Br, I, OH et SH ; où le groupe alkyle est un groupe hydrocarbure de formule générale $C_nH_{2n+1}$, où « n » correspond au nombre d’atomes de carbone et est un entier compris dans la plage de 1 à 18 ; et
où le groupe aryle est un groupe carboné aromatique comprenant 5 à 10 atomes de carbone.

**2.** Réactif solide selon la revendication 1, **caractérisé en ce que** le catalyseur est du nitroprussiate de sodium.

**3.** Réactif solide selon une ou plusieurs des revendications précédentes, **caractérisé en ce que** la base est choisie dans le groupe constitué des phosphates de métal alcalin et/ou alcalino-terreux, des carbonates de métal alcalin et/ou alcalino-terreux et des oxydes de métal alcalin et/ou alcalino-terreux.

**4.** Réactif solide selon une ou plusieurs des revendications précédentes, **caractérisé en ce que** la base est choisie dans le groupe constitué des phosphates de métal alcalin, des carbonates de métal alcalin ou des oxydes de métal alcalino-terreux.

**5.** Réactif solide selon une ou plusieurs des revendications précédentes, **caractérisé en ce que** la source d’hypohalite est choisie dans le groupe constitué de l’acide dichloroisocyanurique, de l’acide trichlorocyanurique et/ou de leurs sels.

**6.** Réactif solide selon une ou plusieurs des revendications précédentes, **caractérisé en ce que** le composé phénolique est choisi dans le groupe constitué du phénol, du thymol, de l’acide salicylique et/ou de leurs sels, du $\alpha$-naphtol, du guajacol, du o-phénylphénol, du o-chlorophénol, de la 2-méthyl-5-hydroxyquinoléine et du m-crésol.

**7.** Réactif solide selon une ou plusieurs des revendications précédentes, **caractérisé en ce que** le réactif solide comprend au moins 2 % en poids de base, par rapport au poids total du réactif séché, et/ou comprend au moins 2 % en poids de composé phénolique, par rapport au poids total du réactif séché.

**8.** Réactif solide selon une ou plusieurs des revendications précédentes, **caractérisé en ce que** le réactif solide comprend jusqu’à 1 % en poids de (i), jusqu’à 2,5 % en poids de (ii), jusqu’à 1 % en poids de (iii) et jusqu’à 25 % en poids de (iv), par rapport au poids total du réactif séché, le reste étant constitué d’additifs ;

jusqu’à 1 % en poids de (i), jusqu’à 5 % en poids de (ii), jusqu’à 1 % en poids de (iii) et jusqu’à 25 % en poids de (iv), par rapport au poids total du réactif séché, le reste étant constitué d’additifs ;
jusqu’à 0,5 % en poids de (i), jusqu’à 55 % en poids de (ii), jusqu’à 0,5 % en poids de (iii) et jusqu’à 15 % en poids de (iv), par rapport au poids total du réactif séché, le reste étant constitué d’additifs ; ou
jusqu’à 1 % en poids de (i), jusqu’à 20 % en poids de (ii), jusqu’à 0,75 % en poids de (iii) et jusqu’à 20 % en poids de (iv), par rapport au poids total du réactif séché, le reste étant constitué d’additifs.

**9.** Réactif solide selon une ou plusieurs des revendications précédentes, **caractérisé en ce que** le réactif est exempt d’eau.

**10.** Réactif solide selon une ou plusieurs des revendications précédentes, dans lequel

(i) le réactif se présente sous forme de sachets de poudre ; ou

(ii) le réactif se présente sous forme de comprimés.

**11.** Kit de test permettant le dosage d'ions ammonium en solution aqueuse, comprenant le réactif solide selon une ou plusieurs des revendications précédentes.

**12.** Utilisation du réactif solide selon une ou plusieurs des revendications 1 à 10 pour une détection qualitative et/ou une détection quantitative d'ions ammonium en solution aqueuse.

**13.** Procédé de détection qualitative d'ions ammonium en solution aqueuse, comprenant les étapes suivantes consistant à :

a) mettre à disposition une solution d'essai ;
b) ajouter le réactif solide selon une ou plusieurs des revendications 1 à 10 à la solution d'essai fournie ou fournir le réactif solide puis ajouter la solution d'essai ;
c) agiter la solution obtenue ;
d) faire incuber dans des conditions normales pendant 30 minutes ou jusqu'à l'apparition d'un changement de couleur de la solution.

**14.** Procédé de détection quantitative d'ions ammonium en solution aqueuse, comprenant les étapes suivantes consistant à :

a) mettre à disposition une solution d'essai ;
b) pipeter la solution d'essai dans une cuvette ;
c) ajouter le réactif solide selon une ou plusieurs des revendications 1 à 10 à la solution d'essai ;
d) dissoudre totalement ou partiellement le réactif solide ajouté ;
e) faire incuber dans des conditions normales pendant 30 min ;
f) déterminer par photométrie l'absorbance de la solution à une longueur d'onde de 680 nm.

**15.** Procédé selon la revendication 13 ou 14, **caractérisé en ce que** l'on utilise entre 10 et 40 mg, et de manière préférée entre 15 et 30 mg, du réactif solide par millilitre de solution d'essai.

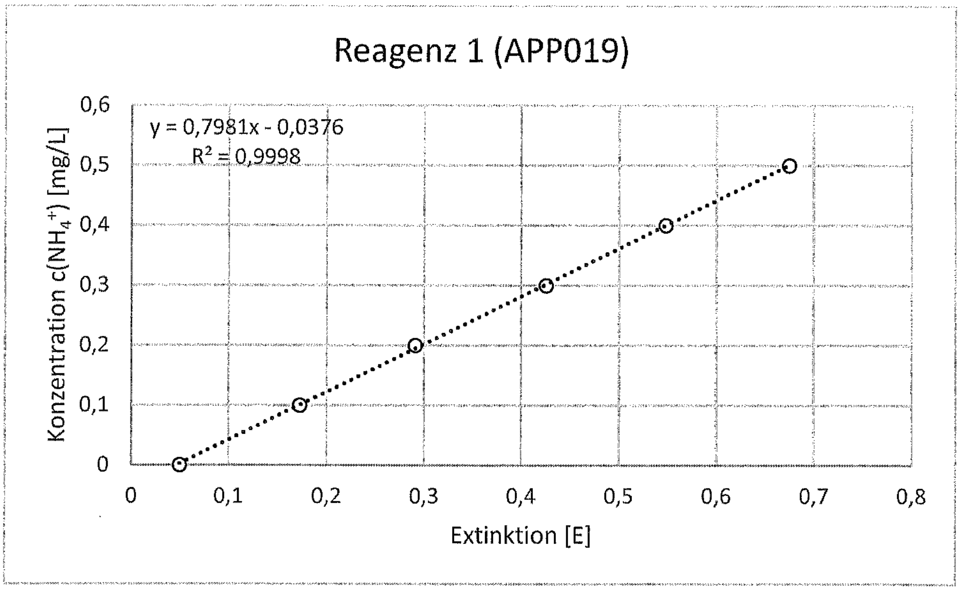


**FIG. 1** *Kalibrierfunktion unter Verwendung von Reagenz 1*

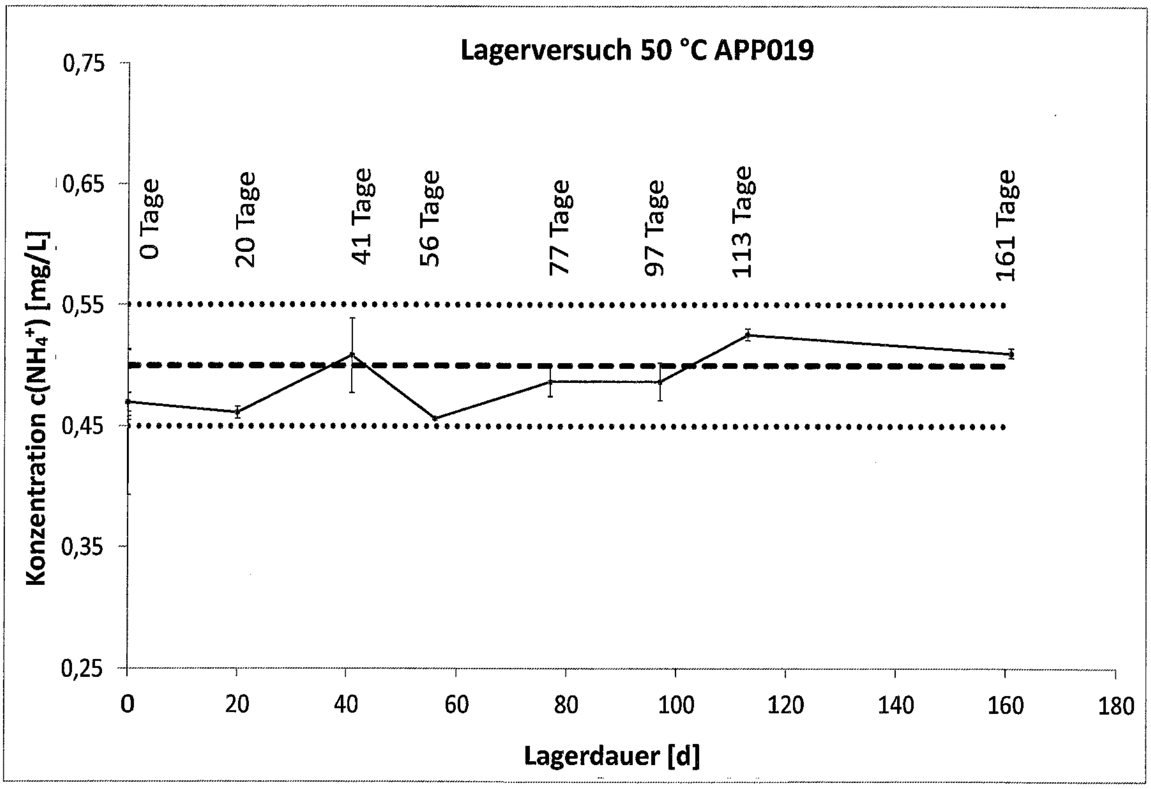


**FIG. 2** *Lagerversuch Reagenz 1*

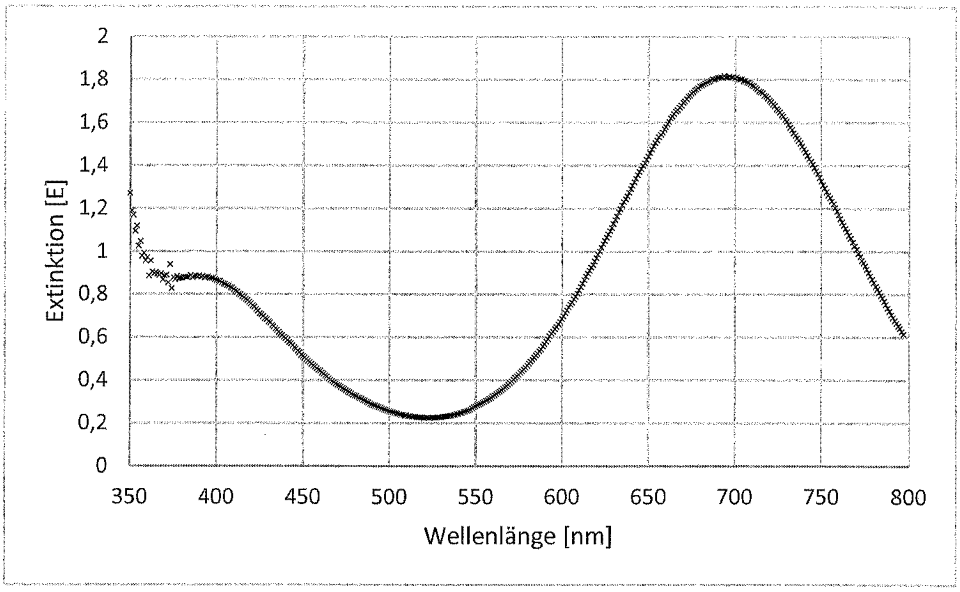


**FIG. 3** *Extinktionsspektrum des gebildeten Indophenolblaus mit Salicylsäure als Phenolverbindung*

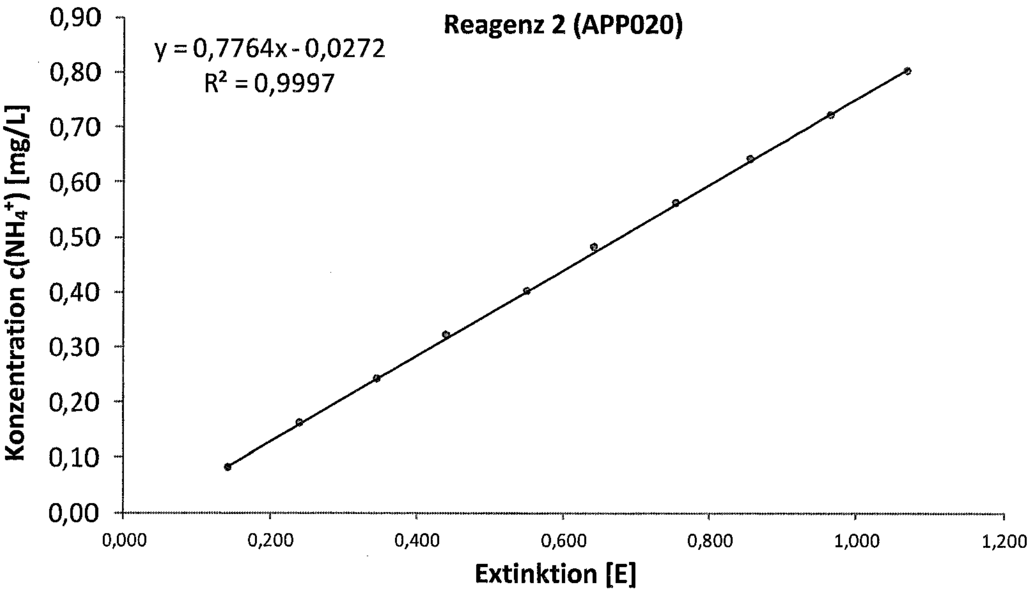


**FIG. 4** Kalibrierfunktion unter Verwendung von Reagenz 2

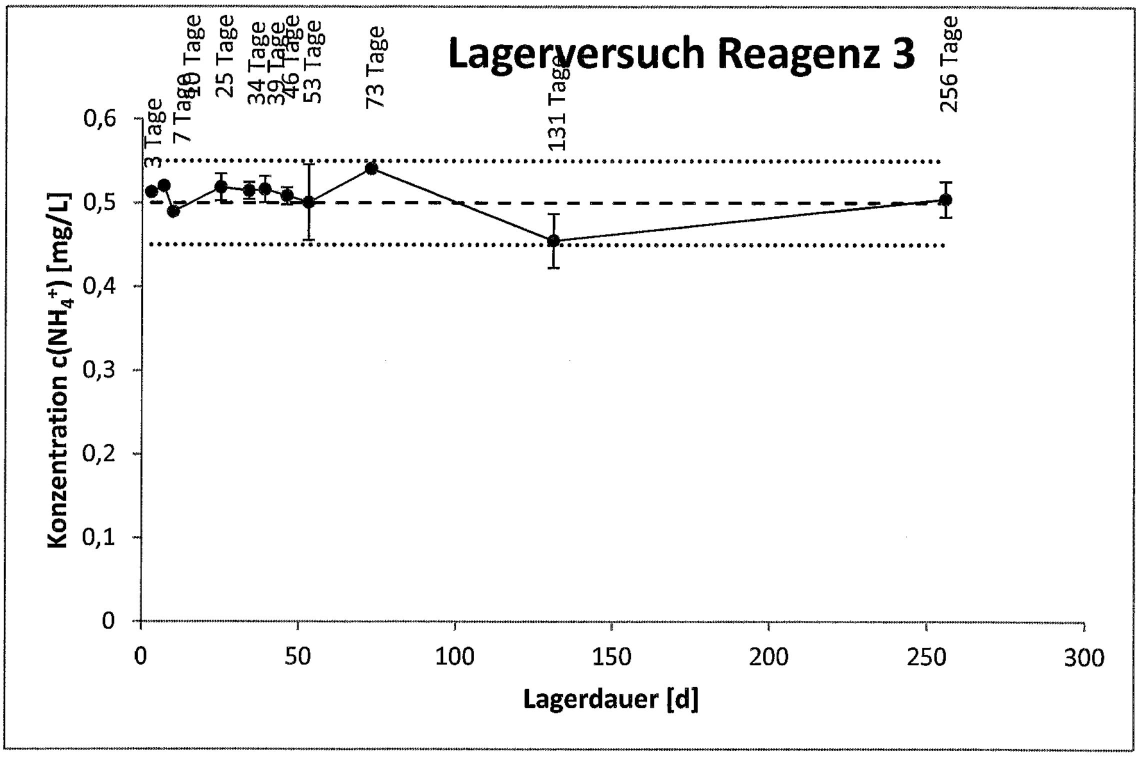


*FIG. 5* Lagerversuch Reagenz 3

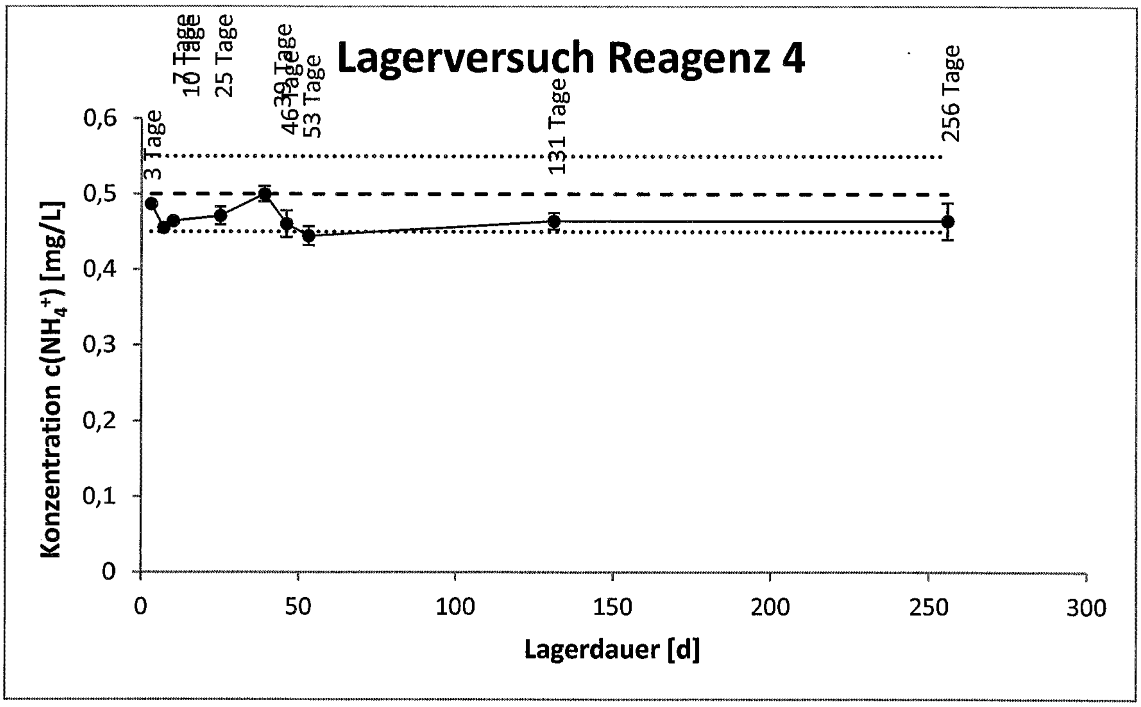


**FIG. 6** *Lagerversuch Reagenz 4*

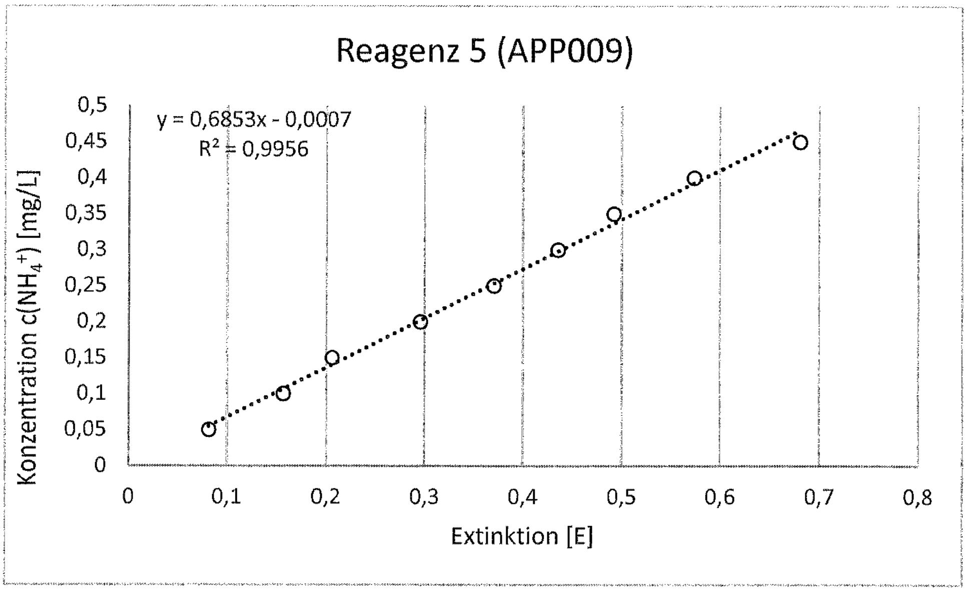


**FIG. 7** *Kalibrierfunktion unter Verwendung von Reagenz 5*

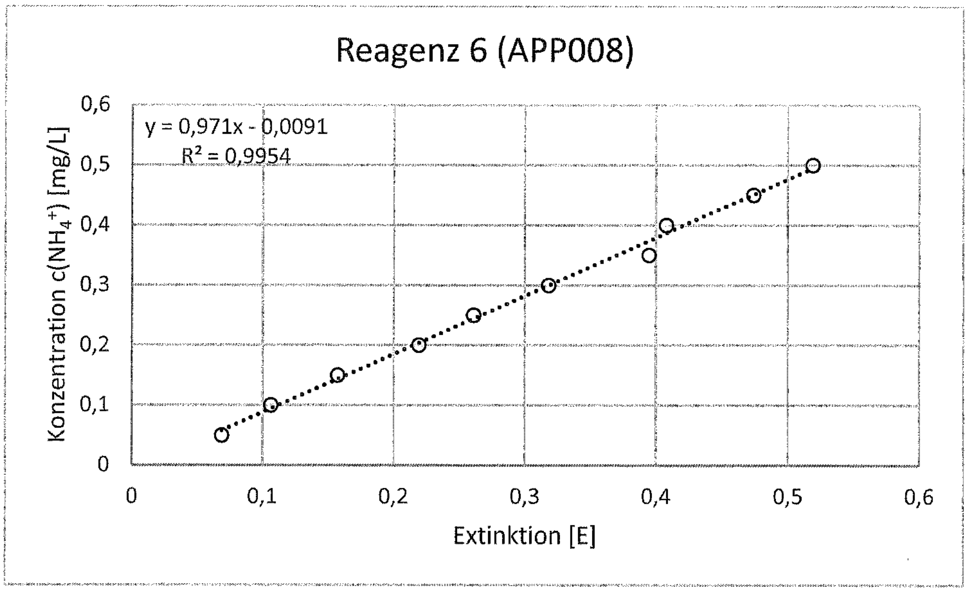


**FIG. 8** *Kalibrierfunktion unter Verwendung von Reagenz 6*

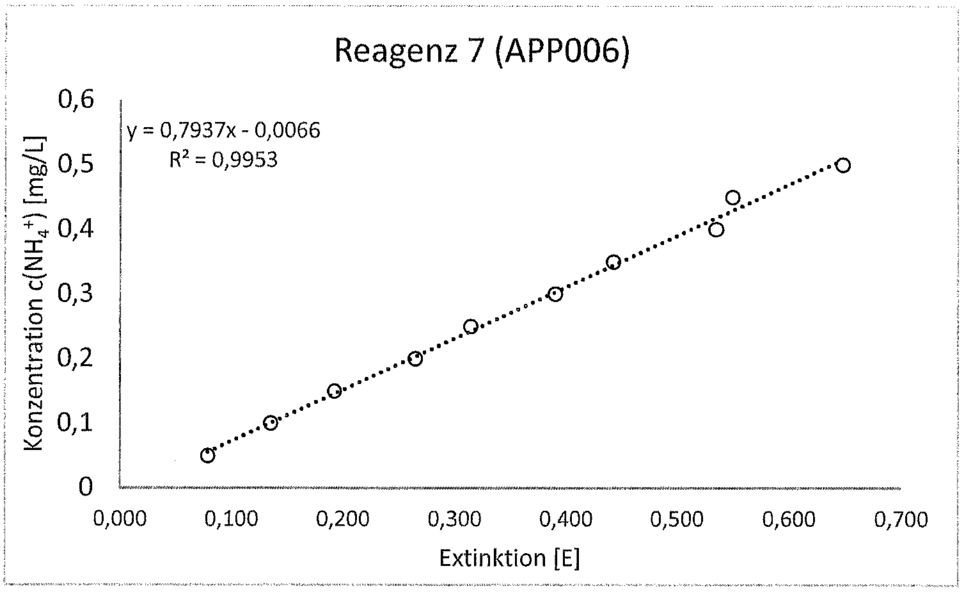


**FIG. 9** *Kalibrierfunktion unter Verwendung von Reagenz 7*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 707210 A
- EP 1840566 A
- WO 2018054797 A
- EP 1566632 A


### In der Beschreibung aufgeführte Nicht-Patentliteratur


- *CHEMICAL ABSTRACTS*, 13755-38-9
- *CHEMICAL ABSTRACTS*, 54-21-7
- *CHEMICAL ABSTRACTS*, 7647-14-5
- *CHEMICAL ABSTRACTS*, 52671-45-1
- *CHEMICAL ABSTRACTS*, 1305-78-8
- *CHEMICAL ABSTRACTS*, 497-19-8
- *CHEMICAL ABSTRACTS*, 554-13-2
- *CHEMICAL ABSTRACTS*, 22763-03-7